(19) Europäisches Patentamt
European Patent Office
Office européen des brevets

(11) EP 3 789 485 A1

(12) EUROPEAN PATENT APPLICATION

(43) Date of publication:
10.03.2021 Bulletin 2021/10

(21) Application number: 19195985.7

(22) Date of filing: 06.09.2019

(51) Int Cl.:
*C12N 5/0783* (2010.01) *A61K 35/17* (2015.01)
*A61K 38/17* (2006.01) *C07K 14/47* (2006.01)
*C07K 14/705* (2006.01)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicant: ONK Therapeutics Limited
Co. Galway H91 V6KV (IE)

(72) Inventor: The designation of the inventor has not yet been filed

(74) Representative: Hughes, Sean David et al
Schlich Limited
9 St. Catherine's Road
Littlehampton, West Sussex BN17 5HS (GB)

# (54) CELLULAR THERAPIES FOR CANCER

(57) NK cells and NK cell lines are modified to increase cytotoxicity, wherein the cells and compositions thereof have a use in the treatment of cancer. Production of modified NK cells and NK cell lines is via genetic modification to express a ligand that binds CD96 and wherein the modified NK cell is not itself bound by the CD96 ligand.

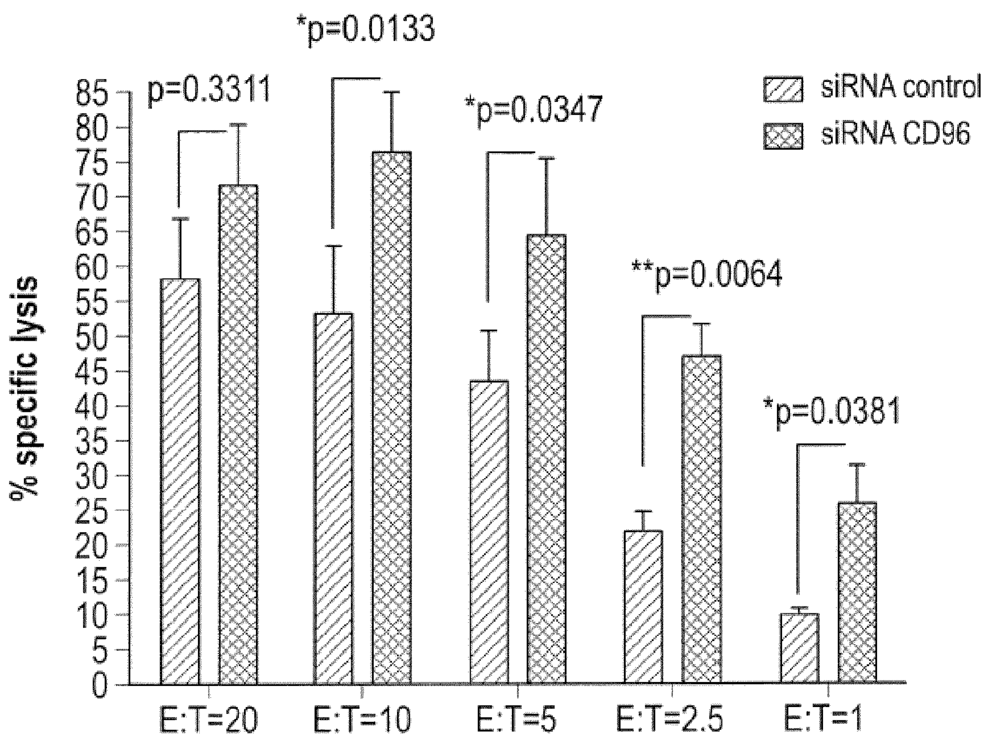


Fig. 2

EP 3 789 485 A1

## Description

### Introduction

The present invention relates to the modification of natural killer (NK) cells and NK cell lines to produce derivatives thereof with improved anti-cancer properties. Furthermore, the present invention relates to methods of producing modified NK cells and NK cell lines, compositions containing the cells and cell lines, and uses of said cells, cell lines and compositions in the treatment of cancer.

### Background to the Invention

Regardless of progress made in the treatment of cancer, there is no long-term cure for numerous cancer patients. In particular, about 70% of acute myeloid leukemia (AML) patients are refractive to treatment. Leukemic stem cells (LSCs) seem to be an important factor in the perpetuation of AML. Most currently used chemotherapeutic agents are not able to eliminate AML-LSCs. In essence, for efficient targeting of AML-LSCs, identification of an appropriate cell surface marker (preferentially expressed on AML-LSCs but absent on normal tissue, especially absent on normal HSCs) is essential. Only a small number of potential target antigens have been identified so far, and unfortunately most of these target antigens are also expressed on normal HSCs or non-hematopoietic tissues.
Cancer stem cells (CSCs) are a small subset of cancer cells that are able to self-renew the cancer stem cell pool (Hope et al., 2004) and lead to relapses in otherwise successfully treated cancer patients. CSCs are highly resistant to conventional therapies which normally target rapidly dividing cells, which explains their contribution to disease relapse (Clarke et al., 2006). Recognition and elimination of the CSC population is therefore crucial in the search for a cure for many cancer patients.
Typically, immune cells require a target cell to present antigen via major histocompatibility complex (MHC) before triggering an immune response resulting in the death of the target cell. This allows cancer cells which downregulate the expression of MHC class I to evade the majority of immune responses including cytotoxic CD8+ T cells.
NK cells are able, however, to recognize cancer cells which have downregulated their MHC class I expression. Hence they perform a critical role in the body's defense against cancer.
In the cancer microenvironment, various immune cells are involved in eliciting an immune response, both individually and as part of a highly complex response involving many different cell types. NK cells, for example, are capable of killing cancer cells directly by way of their specific cytotoxic responses, e.g. perforin and granzyme release. NK cells, however, also attack cancers indirectly via cytokine release.
Cytokines are chemical messengers and have an influence on other cells (Zhang and An, 2007). Two specific cytokines that are fundamental in NK cell based immune responses are interferon $\gamma$ (IFN$\gamma$) and tumour necrosis factor $\alpha$ (TNF$\alpha$). IFN$\gamma$ is considered the prototypic NK cell cytokine that acts on host and tumour cells contributing to anti-tumour effector functions, controlling tumour initiation, growth and metastasis. IFN$\gamma$ upregulates MHC class I as observed in lymphocytes (Boehm et al., 1997), sensitivity to apoptosis as observed in progenitor cells, (Dai et al., 1999) and activates macrophage killing of intracellular pathogens (Caligiuri et al., 2007). Furthermore, the stimulation of NK cells through IFN$\gamma$ is vital in the control of early resistance to infection (Scharton and Scott 1993). Within the immune system, tumour-suppressor function is critically dependent on IFN$\gamma$, since it forms the basis of tumour surveillance.
In certain circumstances, cancer cells demonstrate an ability to dampen the cytotoxic activity of NK cells, through expression of ligands that bind inhibitory receptors on the NK cell membrane. Resistance to cancer can involve a balance between these and other factors.
Cytotoxicity, in this context, refers to the ability of immune effector cells, e.g. NK cells, to induce cancer cell death, e.g. by releasing cytolytic compounds or by binding receptors on cancer cell membranes and inducing apoptosis of said cancer cells. Cytotoxicity is affected not only by signals that induce release of cytolytic compounds but also by signals that inhibit their release. An increase in cytotoxicity will therefore lead to more efficient killing of cancer cells, with less chance of the cancer cell dampening the cytotoxic activity of the NK, as mentioned above.
Several permanent NK cell lines have been established, and the most notable is NK-92, derived from a patient with non-Hodgkin's lymphoma expressing typical NK cell markers, with the exception of CD16 (Fc gamma receptor IIIa). NK-92 has undergone extensive preclinical testing and exhibits superior lysis against a broad range of tumours compared with activated NK cells and lymphokine-activated killer (LAK) cells (Gong, Maki et al. 1994). Cytotoxicity of NK-92 cells against primary AML has been established (Yan, Steinherz et al. 1998).
Another NK cell line, KHYG-1, has been identified as a potential contender for clinical use (Suck et al. 2005). KHYG-1 cells are known to be pre-activated. Unlike endogenous NK cells, KHYG-1 cells are polarized at all times, increasing their cytotoxicity and making them quicker to respond to external stimuli. NK-92 cells have a higher baseline cytotoxicity than KHYG-1 cells.
It is clear that current adoptive immunotherapy protocols are affected by donor variability in the quantity and quality of effector cells, variables that could be eliminated if effective cell lines were available to provide more standardized therapy.

Mouse and human NK cells vary greatly in their expression markers, signaling cascades and tissue distribution. For example, CD56 is used as a marker for human NK cells, whereas mouse NK cells do not express this marker at all. Furthermore, a well-established mechanism for regulating NK cell cytotoxicity is via ligand binding NK activation and inhibitory receptors. Two of the most prominent human NK activation receptors are known to be NKp30 and NKp44, neither of which are expressed on mouse NK cells. With regards to NK inhibitory receptors, whilst human NK cells express KIRs that recognise MHC class I and dampen cytotoxic activity, mouse NK cells do not express KIRs at all but, instead, express Ly49s (Trowsdale et al, 2001). All in all, despite mouse NK cells achieving the same function as human NK cells in their natural physiological environment, the mechanisms that fulfil this role vary significantly between species.

Thus there exists a need for alternative and preferably improved human NK cells and human NK cell lines that are better able to target cancer cells *in vivo.*

An object of the invention is to provide NK cells and NK cell lines with an improved anti-cancer phenotype, including improved NK cell-mediated cytotoxicity and / or improved cytokine production. A further object is to provide methods for producing modified NK cells and NK cell lines, compositions containing the cells or cell lines and uses of said compositions in the treatment of cancers. More particular embodiments aim to provide treatments for identified cancers, e.g. blood cancers, such as leukemias. Specific embodiments aim at combining two or more modifications of NK cells and NK cell lines to further enhance the anti-cancer activity of the modified cells.

Specifically, one problem that is common when targeting a specific cancer antigen is that the same antigen is present on normal tissue as well. This results in characteristic on-target off-tumour side-effects. A lesser known problem, however, is the use of cells in cancer therapy that also express the antigen. It is generally considered very problematic for the therapeutic cells to target an antigen that they themselves express, since this phenomenon invariably leads to cellular fratricide and hence a non-viable cancer therapy.

A specific aim of the present invention is therefore to provide an NK cell that selectively binds cancer cells, e.g. AML cells, whilst avoiding NK cell fratricide.

## Summary of the Invention

There are provided herein modified NK cells and NK cell lines with improved anti-cancer properties and methods of making the cells and cell lines. Also provided are compositions of modified NK cells and NK cell lines, and uses of said cells, cell lines and compositions for treating cancer.

The invention provides methods of modifying NK cells and NK cell lines using, for example, genetic engineering to knock down / knock out CD96 and also express genes encoding CD96 chimeric antigen receptors (CARs).

Furthermore, compositions of the invention include NK cells and NK cell lines in which two or more modifications are provided, wherein multiple modifications further enhance the anti-cancer activity of the composition.

According to the invention, there are further provided methods of treating cancer, e.g. blood cancer, using modified NK cells and cell lines, e.g. derivatives of KHYG-1 cells, wherein the modified NK cells and cell lines are engineered to express a ligand that binds CD96 (e.g. CD155, CD111 and/or CD112) and wherein the modified NK cell is not itself bound by CD96 ligand.

Diseases particularly treatable according to the invention include cancers, blood cancers, leukemias and specifically acute myeloid leukemia. Tumours and cancers in humans in particular can be treated. References to tumours herein include references to neoplasms.

## Details of the Invention

Accordingly, the present invention provides a natural killer (NK) cell or NK cell line, wherein the NK cell or NK cell line expresses a ligand that binds CD96 and wherein the NK cell or NK cell line is not itself bound by the CD96 ligand.

For the avoidance of doubt, the term NK cell should be construed to encompass any NK cell, NK cell line, or derivative thereof. NK cells can be expanded under suitable growth conditions for therapeutic use.

It is preferred that the NK cell has been modified to express a chimeric antigen receptor (CAR) for CD96. The CAR can be a bispecific CAR, i.e. capable of binding two specific targets. In addition to binding CD96, the bispecific CAR preferably also binds CD38, CD33, DR4 or DR5. It is preferred that both target antigens are expressed on the cancer cell. Preferably, the bispecific CAR binds CD96 and CD38.

Preferably, the CAR is linked to a co-stimulatory domain, e.g. 41BB (CD137), CD28, OX40, CD3zeta (CD247), 2B4, DAP12 and/or DAP10.

Optionally, the NK cell has been modified to express a natural ligand for CD96, e.g. CD155 or CD111. It is preferred in this case that the natural CD96 ligand is modified to have higher affinity for CD96 than the wildtype form of the natural ligand. Preferably, the NK cell has been modified to express a high-affinity variant of CD155.

By using the term "high-affinity" herein, it is meant that the modified ligand (be it natural, chimeric or other) has a higher affinity for its target than the same ligand unmodified. For example, CD155 (ligand) naturally binds CD96 (receptor) with a given affinity (conventionally determined using a number of assays known to the skilled person, e.g. surface plasmon resonance, isothermal titration calorimetry or ELISA). If CD155 is modified to bind CD96 with a higher affinity, CD155 binds CD96 with a higher affinity relative to the unmodified version.

Preferably, the modified "high-affinity" ligand binds its target with at least 25% higher affinity than the unmodified version of the ligand. Preferably, affinity is increased by at least 50%, more preferably increased by at least 100%, more preferably increased by at least 200%, and most preferably increased by at least 500%.

It is preferred that the NK cell according to the invention does not itself express CD96. This advantageously makes the NK cell less susceptible to fratricide resulting from the expression of ligands, CARs etc. that bind CD96.

Preferably, the NK cell is modified to have knocked down or knocked out expression of CD96. The terms genetic knockdown and genetic knockout are well known to the skilled person. Nevertheless, for clarity, an NK cell with CD96 knocked out is defined as an NK cell that expresses substantially no CD96, whereas an NK cell with CD96 knocked down is defined as an NK cell that still expresses a low level of CD96 compared to the wildtype version of the NK cell.

Preferably, if a gene (e.g. CD96 or TIGIT) is knocked down, the expression of that gene in the cell is reduced by at least 10% compared to the unmodified version (i.e. wildtype cell). Preferably, expression is reduced by at least 25%, more preferably reduced by at least 50%, more preferably reduced by at least 75%, and most preferably reduced by at least 90%.

In a preferred embodiment of the invention, CD96 and/or TIGIT expression in the NK cell is reduced by at least 80% compared to a wildtype NK cell.

Preferably, the NK cell has been modified using the CRISPR gene editing method. CRISPR gene editing allows for genes to be knocked in, knocked down and knocked out. Optionally, however, RNAi (e.g. siRNA or shRNA) is used to modify the NK cell.

The invention also provides NK cells that have been modified to express CD96 linked to a co-stimulatory domain. Naturally, CD96 is a known checkpoint inhibitory receptor and acts to dampen cytotoxic responses in NK cells. By modifying CD96 to be linked to a co-stimulatory domain, it is possible to convert the inhibitory signal into an activating signal, making the NK cell more cytotoxic. Preferably, the co-stimulatory domain is selected from 41BB (CD137), CD28, OX40, CD3zeta (CD247), 2B4, DAP12 and/or DAP10.

The invention also provides NK cells that have been modified to express TIGIT linked to a co-stimulatory domain. Naturally, TIGIT is a known checkpoint inhibitory receptor and acts to dampen cytotoxic responses in NK cells. By modifying TIGIT to be linked to a co-stimulatory domain, it is possible to convert the inhibitory signal into an activating signal, making the NK cell more cytotoxic. Preferably, the co-stimulatory domain is selected from 41BB (CD137), CD28, OX40, CD3zeta (CD247), 2B4, DAP12 and/or DAP10.

In optional embodiments of the invention, the NK cell has been further modified to have reduced expression of one or more additional checkpoint inhibitory receptors. Preferably, the checkpoint inhibitory receptor is selected from CD152 (CTLA4), CD223 (LAG-3), CD279 (PD-1), CD328 (SIGLEC7), SIGLEC9, TIGIT and TIM-3. This has the advantageous effect of making the NK cell more cytotoxic in general, specifically in the tumour microenvironment.

It is preferred that expression of TIGIT is either knocked down or knocked out, since TIGIT is known to compete with CD96 for binding of e.g. CD155.

The NK cell may optionally also have been modified to express a mutant form of TRAIL ligand (e.g. 4C9 or E195R/D269H), wherein the mutant TRAIL ligand has an increased affinity for one or more TRAIL death receptors, e.g. DR4 and/or DR5. This has the advantageous effect of making the NK cell more cytotoxic against a range of cancers expressing TRAIL death receptors.

In an optional embodiment of the invention, CD96 function on NK cells is reduced or further reduced by blocking CD96 using an antagonist. This antagonist may be e.g. a small molecule antagonist, a neutralizing antibody, a $F(ab)_2$ fragment etc.

In one preferred embodiment of the invention, the NK cell is modified to (1) express a CAR for CD96, (2) have CD96 expression knocked down, and (3) have TIGIT expression knocked down. Preferably, the NK cell also expresses mutant TRAIL ligand. Preferably, the mutant TRAIL ligand has increased affinity for DR4. Preferably, the NK cell is further modified to express a second CAR for a different cancer antigen (e.g. CD38 or CD33).

The NK cell according to the invention may be an autologous NK cell, an allogenic NK cell (e.g. donor-derived, cord blood-derived or iPSC-derived), or an NK cell line. Preferably, the NK cell is a KHYG-1 cell or a derivative thereof.

In another embodiment of the invention, there is provided an NK cell according to the invention (described above and below), wherein that NK cell is for use in treating cancer. Preferably, the cancer is human cancer.

It is preferred that the NK cell is for use in treating a CD96-expressing cancer.

Preferably, the cancer is a blood cancer. Preferably, the blood cancer is a leukemia, e.g. acute lymphocytic leukemia (ALL), T-cell acute lymphoblastic leukemia (T-ALL) or acute myeloid leukemia (AML). Most preferably, the cancer is AML.

The NK cells of the invention are also useful in the treatment of stem cell cancers. In particular, the NK cells are capable of killing leukemic stem cells. It has been found that AML-LSCs express CD96, and thus CD96 is considered a good candidate for targeting this group of AML cells that are thought to be so instrumental in the relapse of as many as 70% of AML patients.

The invention hence further provides a method of treating cancer, the method comprising administering to a patient an NK cell or NK cell line, wherein the NK cell or NK cell line expresses a ligand that binds CD96 and wherein the NK cell or NK cell line is not itself bound by the CD96 ligand. Preferably, the NK cell or NK cell line is administered to the patient in combination a pharmaceutically acceptable excipient. Preferably, the patient is a human.

Preferably, the method comprises administering to the patient a modified NK cell according to the invention, as described in detail above and below.

As such, the invention further provides a pharmaceutical composition comprising an NK cell according to the invention, as described in detail above and below. The pharmaceutical composition comprising the NK cell is suitable for use in treating cancer as described in detail above and below.

The invention also provides a composition comprising:

a CRISPR-Cas (CRISPRCas) system RNA polynucleotide sequence, wherein the polynucleotide sequence comprises

(a) a guide sequence of between 10 - 30 nucleotides in length, capable of hybridizing to the CD96 sequence in a eukaryotic cell,
(b) a tracr mate sequence, and
(c) a tracrRNA sequence

wherein, optionally when transcribed, the tracr mate sequence hybridizes to the tracrRNA sequence and the guide sequence directs sequence-specific binding of a CRISPR complex to the target sequence,
wherein the CRISPR complex comprises a Type II Cas9 protein complexed with (1) the guide sequence that is hybridized to the CD96 sequence, and (2) the tracr mate sequence that is hybridized to the tracrRNA sequence.

The invention also provides a composition comprising:

a CRISPR-Cas (CRISPRCas) system RNA polynucleotide sequence, wherein the polynucleotide sequence comprises

(a) a guide sequence of between 10 - 30 nucleotides in length, capable of hybridizing to the CD96 sequence in a eukaryotic cell,
and
(b) a tracrRNA sequence or
(c) a chimeric guide which combines the guide with tracrRNA,

wherein the tracrRNA-guide sequence(s) complex with Cas9 to the CD96 sequence, and cause a mutation, e.g. one or more subsequent indels, in the CD96 gene (e.g. in exon 1, exon 2, or exon 3).

A variant, wherein a pool of multiple tracrRNA-guides is used, targeting different portions of the CD96 sequence, can be used to complete deletion of 10-20 bp nucleotide sequences, thereby generating CD96 Knockout cells.

It is preferred that the composition is a non-naturally occurring or engineered composition. It is further preferred that the tracrRNA sequence is 50 or more nucleotides in length.

Optionally, the RNA is chimeric RNA (chiRNA). In this case, it is preferred that (a), (b) and (c) are arranged in a 5' to 3' orientation.

The invention also provides a method of making a modified NK cell comprising a CD96 knockdown or knockout using the composition described above.

Preferably, a gene encoding a CD96-CAR (e.g. SEQ ID NO:1) is inserted at the location of the DNA double-strand break. As such, it is preferred that CD96 gene expression is knocked down using the same composition that knocks in the CD96-CAR. Optionally, the CAR is linked to a co-stimulatory domain, e.g. 41BB (CD137), CD28, OX40, CD3zeta (CD247), DAP12 and/or DAP10. Optionally, the CAR is bispecific.

## Examples

The present invention is now described in more and specific details in relation to the production of NK cell line KHYG-1 derivatives, modified to exhibit increased anti-cancer activity and hence ability to cause leukemia cell death in humans.

The invention is now illustrated in specific embodiments with reference to the accompanying drawings in which:

Fig. 1A is a FACS plot showing successful CD96 knockdown using siRNA-based gene silencing;
Fig. 1B is a FACS plot showing successful CD96 knockdown using siRNA-based gene silencing;
Fig. 2 is a bar chart showing increased cytotoxicity of CD96 knockdown KHYG-1 cells against leukemia cells at various E:T ratios;
Fig. 3 shows FACS plots of apoptosis in leukemia cells co-cultured with KHYG-1 cells with CD96-siRNA and/or TRAIL variant expression;
Fig. 4 shows FACS plots of apoptosis in multiple myeloma cells co-cultured with KHYG-1 cells with CD96-siRNA and/or TRAIL variant expression; and
Fig. 5 is a FACS plot showing successful CD96 knockdown using CRISPR-based gene editing;
Fig. 6 shows bar charts of the effect CD96 knockdown in NK cells has on IFNy secretion in the presence / absence of multiple cancer cell lines; and
Fig 7 shows bar charts of the effect CD96 knockdown in NK cells has on TNF$\alpha$ secretion in the presence / absence of multiple cancer cell lines.

DNA, RNA and amino acid sequences are referred to below, in which:

SEQ ID NO: 1 is the CD96 CAR DNA sequence; and
SEQ ID NO: 2 is the CD96 CAR peptide sequence.

<u>SEQ ID NO: 1</u>

ATGTGGCAACTGCTGCTGCCTACAGCTCTGCTGCTTCTGGTGTCCGCCGCCAT
GGCGGACTACAAAGACATTGTGCTGACCCAATCTCCAGCTTCTTTGGCTGTGTC
TCTAGGGCAGAGGGCCACCATATCCTGCAGAGCCAGTGAAAGTGTTGATAGTTA
TGGCAATAGTTTTATGCACTGGTACCAGCAGAAACCAGGACAGCCACCCAAACT
CCTCATCTATCGTGCATCCAATTTAGAATCTGGGATCCCTGCCAGGTTCAGTGG
CAGTGGGTCTAGGACAGACTTCACCCTCACCATTAATCCTGTGGAGGCTGATGA
TGTTGCAACCTATTACTGTCAGCAAAGTAATGAGGATCCGTACACGTTCGGAGG
GGGGACCAAGCTGGAAATAAAACGTGGTGGTGGTGGTTCTGGTGGTGGTGGTT
CTGGCGGCGGCGGCTCCGGTGGTGGTGGATCCGAGGTGCAGCTGAAGGAGTC
TGGCCCTGGGATATTGCAGCCCTCCCAGACCCTCAGTCTGACTTGTTCCTTCTC
TGGGTTTTCACTGAACACCTCTGGTATGGGTGTAGGCTGGATTCGTCAGCCTTC
AGGGAAGGGTCTGGAGTGGCTGGCACACATTTGGTGGGATGATGACAAGCGCT
ATAACCCAGCCCTGAAGAGCCGACTGACATTCTCCAAGGATACCTCCAGCAACC

AGGTATTCCTCAAGATCGCCAGTGTGGACACTGCAGATACTGCCACATACTACT
GTGTTCGAGGGGGTAACTCGTTTGCTTACTGGGGCCAAGGGACTCTGGTCACT
GTCTCTACCTTCACCTGTTTTGTCGTGGGCAGCGACCTGAAGGATGCCCACCTG
ACATGGGAAGTCGCCGGCAAAGTTCCTACCGGTGGCGTGGAAGAAGGCCTGCT
GGAAAGACACAGCAACGGCAGCCAGAGCCAGCACAGCAGACTGACACTGCCTA
GAAGCCTGTGGAATGCCGGCACCAGCGTGACCTGCACACTGAATCATCCTAGC
CTGCCTCCACAGAGACTGATGGCCCTGAGAGAACCTGCTGCTCAGGCCCCTGT
GAAGCTGTCCCTGAATCTGCTCGCCAGCAGCGATCCTCCTGAAGCCGCCAATG
TGAACCACAAGCCTAGCAACACCAAGGTGGACAAGAAGGTGGAACCCAAGAGC
TGCGACAAGACCCACACCTGTCCTCCATGTCCTGCTCCAGAACTGCTCGGCGG
ACCTTCCGTGTTCCTGTTTCCTCCAAAGCCTAAGGACACCCTGATGATCAGCAG
AACCCCTGAAGTGACCTGCGTGGTGGTGGATGTGTCCCACGAGGATCCCGAAG
TGAAGTTCAATTGGTACGTGGACGGCGTCGAGGTGCACAACGCCAAGACAAAG
CCCAGAGAGGAACAGTACAACAGCACCTACAGAGTGGTGTCCGTGCTGACCGT
GCTGCACCAGGATTGGCTGAACGGCAAAGAGTACAAGTGCAAGGTGTCCAACA
AGGCCCTGCCTGCTCCTATCGAGAAAACCATCAGCAAGGCCAAGGGCCAGCCT
AGAGAACCCCAGGTGTACACACTGCCTCCAAGCAGAGATGAGCTGACCAAGAA
CCAGGTGTCCCTGACATGCCTGGTCAAGGGCTTCTACCCCTCCGATATCGCCG
TGGAATGGGAGAGCAATGGACAGCCCGAGAACAACTACAAGACAACCCCTCCT
GTGCTGGACTCCGACGGCTCATTCTTCCTGTACAGCAAACTGACCGTGGACAA
GTCCAGATGGCAGCAGGGCAACGTGTTCTCCTGCAGCGTGATGCACGAGGCCC
TGCACTTTTGGGTGCTCGTGGTTGTTGGCGGAGTGCTGGCCTGTTACAGCCTG
CTGGTTACCGTGGCCTTCATCATCTTTTGGGTCCGAAGCAAGCGGAGCCGGCT
GCTGCACAGCGACTACATGAACATGACCCCTAGACGGCCCGGACCTACCAGAA
AGCACTACCAGCCTTACGCTCCTCCTAGAGACTTCGCCGCCTACAGAAGCAGA
CGGGACCAAAGACTGCCTCCTGACGCTCACAAACCTCCAGGCGGCGGAAGCTT
CAGGACCCCTATCCAAGAAGAACAGGCTGACGCCCACAGCACCCTGGCCAAGA
TCCGCGTGAAGTTCTCCAGATCCGCCGACGCTCCTGCCTATCAGCAGGGACAG
AACCAGCTGTACAACGAGCTGAACCTGGGGAGAAGAGAAGAGTACGACGTGCT
GGATAAGCGGAGAGGCAGAGATCCTGAGATGGGCGGAAAGCCCCAGCGGAGA
AAGAATCCTCAAGAGGGCCTGTATAATGAGCTGCAGAAAGACAAGATGGCCGA
GGCCTACAGCGAGATCGGAATGAAGGGCGAGCGCAGAAGAGGCAAGGGACAC
GATGGACTGTACCAGGGACTGAGCACCGCCACCAAGGATACCTATGACGCCCT
GCACATGCAGGCTCTGCCTCCA

SEQ ID NO: 2

MWQLLLPTALLLLVSAAMADYKDIVLTQSPASLAVSLGQRATISCRASESVDSYGNS
FMHWYQQKPGQPPKLLIYRASNLESGIPARFSGSGSRTDFTLTINPVEADDVATYY
CQQSNEDPYTFGGGTKLEIKRGGGGSGGGGSGGGGSGGGGSEVQLKESGPGILQ
PSQTLSLTCSFSGFSLNTSGMGVGWIRQPSGKGLEWLAHIWWDDDKRYNPALKSR
LTFSKDTSSNQVFLKIASVDTADTATYYCVRGGNSFAYWGQGTLVTVSTFTCFVVG
SDLKDAHLTWEVAGKVPTGGVEEGLLERHSNGSQSQHSRLTLPRSLWNAGTSVTC
TLNHPSLPPQRLMALREPAAQAPVKLSLNLLASSDPPEAANVNHKPSNTKVDKKVE
PKSCDKTHTCPPCPAPELLGGPSVFLFPPKPKDTLMISRTPEVTCVVVDVSHEDPE
VKFNWYVDGVEVHNAKTKPREEQYNSTYRVVSVLTVLHQDWLNGKEYKCKVSNK
ALPAPIEKTISKAKGQPREPQVYTLPPSRDELTKNQVSLTCLVKGFYPSDIAVEWES
NGQPENNYKTTPPVLDSDGSFFLYSKLTVDKSRWQQGNVFSCSVMHEALHFWVLV
VVGGVLACYSLLVTVAFIIFWVRSKRSRLLHSDYMNMTPRRPGPTRKHYQPYAPPR
DFAAYRSRRDQRLPPDAHKPPGGGSFRTPIQEEQADAHSTLAKIRVKFSRSADAPA
YQQGQNQLYNELNLGRREEYDVLDKRRGRDPEMGGKPQRRKNPQEGLYNELQK
DKMAEAYSEIGMKGERRRGKGHDGLYQGLSTATKDTYDALHMQALPP

**Example 1 - Knockdown of Checkpoint Inhibitory Receptor CD96 Function via RNAi**

siRNA knockdown of CD96 in KHYG-1 cells was performed by electroporation. The Nucleofection Kit T was used, in conjunction with the Amaxa Nucleofector II, from Lonza, as it is appropriate for use with cell lines and can successfully transfect both dividing and non-dividing cells and achieves transfection efficiencies of up to 90%..

Control siRNA (catalog number: sc-37007) and CD96 siRNA (catalog number: sc-45460) were obtained from Santa Cruz Biotechnology. Antibiotic-free RPMI-1640 containing 10% FBS, 2mM L-glutamine was used for post-Nucleofection culture. Mouse anti-human CD96-APC (catalog number: 338409) was obtained from Biolegend for staining.

A 20μM of siRNA stock solution was prepared. The lyophilized siRNA duplex was resuspended in 33μl of the RNAse-free water (siRNA dilution buffer: sc-29527) to FITC-control/control-siRNA, in 165μl of the RNAse-free water for the target gene siRNA (siRNA CD96). The tube was heated to 90°C for 1 minute and then incubated at 37°C for 60 minutes. The siRNA stock was then stored at -20°C until needed. The KHYG-1 cells were passaged one to two days before Nucleofection, as the cells must be in logarithmic growth phase.

The Nucleofector solution was warmed to room temperature (100ul per sample). An aliquot of culture medium containing serum and supplements was also pre-warmed at 37°C in a 50ml tube. 6-well plates were prepared by adding 1.5ml of culture medium containing serum and supplements. The plates were pre-incubated in a humidified 37°C / 5% $CO_2$ incubator.

$2x10^6$ cells in 100μl Nucleofection solution was mixed gently with 4μl 20μM siRNA solution (1.5μg siRNA). Air bubbles were avoided during mixing. The mixture was transferred into Amaxa certified cuvettes and placed into the Nucleofector cuvette holder and program U-001 selected.

The program was allowed to finish, and the samples in the cuvettes were removed immediately. 500μl pre-equilibrated culture medium was then added to each cuvette. The sample in each cuvette was then gently transferred to a corresponding well of the prepared 6-well plate, in order to establish a final volume of 2ml per well.

The cells were then incubated in a humidified 37°C / 5% $CO_2$ incubator until transfection analysis was performed. Flow cytometry analysis was performed 16-24 hours after electroporation, in order to measure CD96 expression levels. This electroporation protocol was carried out multiple times and found to reliably result in CD96 knockdown in KHYG-1 cells (see e.g. Figures 1A and 1B).

**Example 2 - Enhanced Cytotoxicity of NK Cells with a CD96 Knockdown**

KHYG-1 cells with and without the CD96 knockdown were co-cultured with K562 cells at different effector:target (E:T) ratios.

Cytotoxicity was measured 4 hours after co-culture, using the DELFIA EuTDA Cytotoxicity Kit from PerkinElmer (Catalog number: AD0116).

Target cells K562 were cultivated in RPMI-1640 medium containing 10% FBS, 2mM L-glutamine and antibiotics. 96-well V-bottom plates (catalog number: 83.3926) were bought from SARSTEDT. An Eppendorf centrifuge 5810R (with plate rotor) was used to spin down the plate. A VARIOSKAN FLASH (with ScanIt software 2.4.3) was used to measure the fluorescence signal produced by lysed K562 cells.

K562 cells were washed with culture medium and the number of cells adjusted to $1x10^6$ cells/mL with culture medium. 2-4mL of cells was added to 5μl of BATDA reagent and incubated for 10 minutes at 37°C. Within the cell, the ester bonds are hydrolysed to form a hydrophilic ligand, which no longer passes through the membrane. The cells were centrifuged at 1500RPM for 5 mins to wash the loaded K562 cells. This was repeated 3-5 times with medium containing 1mM Probenecid (Sigma P8761). After the final wash the cell pellet was resuspended in culture medium and adjusted to about $5\ x10^4$ cells/mL.

Wells were set up for detection of background, spontaneous release and maximum release. 100μL of loaded target cells (5,000 cells) were transferred to wells in a V-bottom plate and 100μL of effector cells (KHYG-1 cells) were added at varying cell concentrations, in order to produce effector to target ratios ranging from 1:1 to 20:1. The plate was centrifuged at 100xg for 1 minute and incubated for 4 hours in a humidified 5% $CO_2$ atmosphere at 37°C. For maximum release wells 10μL of lysis buffer was added to each well 15 minutes before harvesting the medium. The plate was centrifuged at 500xg for 5 minutes.

20μL of supernatant was transferred to a flat-bottom 96 well plate 200μL of pre-warmed Europium solution added. This was incubated at room temperature for 15 mins using a plate shaker. As K562 cells are lysed by the KHYG-1 cells, they release ligand into the medium. This ligand then reacts with the Europium solution to form a fluorescent chelate that directly correlates with the amount of lysed cells.

The fluorescence was then measured in a time-resolved fluorometer by using VARIOSKAN FLASH. The specific release was calculated using the following formula:

% specific release = Experiment release – Spontaneous release / Maximum release – Spontaneous release

Statistical analysis was performed using Graphpad Prism 6.04 software. A paired t test was used to compare the difference between siRNA CD96 knockdown KHYG-1 cells and control groups (n=3).

The specific release was found to be significantly increased in co-cultures containing the CD96 knockdown KHYG-1 cells. This was the case at all E:T ratios (see Figure 2).

As fluorescence directly correlates with cell lysis, it was confirmed that knocking down CD96 expression in KHYG-1 cells resulted in an increase in their ability to kill K562 cancer target cells.

**Example 3 - Knock-in of TRAIL / TRAIL Variant in NK Cells**

KHYG-1 cells were transfected with both TRAIL and TRAIL variant, in order to assess their viability and ability to kill cancer cells following transfection.

The TRAIL variant used is that described in WO 2009/077857. It is encoded by the wildtype TRAIL gene containing the D269H/E195R mutation. This mutation significantly increases the affinity of the TRAIL variant for DR5, whilst reducing the affinity for both decoy receptors (DcR1 and DcR2).

<u>Baseline TRAIL Expression</u>

Baseline TRAIL (CD253) expression in KHYG-1 cells was assayed using flow cytometry.

Mouse anti-human CD253-APC (Biolegend catalog number: 308210) and isotype control (Biolegend catalog number: 400122) were used to stain cell samples and were analyzed on a BD FACS Canto II flow cytometer.

KHYG-1 cells were cultured in RPMI 1640 medium containing 10% FBS, 2mM L-glutamine, penicillin (100 U/mL)/streptomycin (100 mg/mL) and IL-2 (10ng/mL). 0.5-1.0 $\times 10^6$ cells/test were collected by centrifugation (1500rpm x 5 minutes) and the supernatant was aspirated. The cells (single cell suspension) were washed with 4 mL ice cold FACS Buffer (PBS, 0.5-1 % BSA, 0.1% NaN3 sodium azide). The cells were re-suspended in 100 μL ice cold FACS

Buffer, add 5uL antibody was added to each tube and incubated for 30 minutes on ice. The cells were washed 3 times by centrifugation at 1500 rpm for 5 minutes. The cells were then re-suspended in 500 μL ice cold FACS Buffer and temporarily kept in the dark on ice.

The cells were subsequently analyzed on the flow cytometer (BD FACS Canto II) and the generated data were processed using FlowJo 7.6.2 software. FACS analysis showed weak baseline expression of TRAIL on the KHYG-1 cell surface.

TRAIL / TRAIL variant Knock-in by Electroporation

Wildtype TRAIL mRNA and TRAIL variant (D269H/195R) mRNA was synthesized by TriLink BioTechnologies, aliquoted and stored as -80°C. Mouse anti-human CD253-APC (Biolegend catalog number: 308210) and isotype control (Biolegend catalog number: 400122), and Mouse anti-human CD107a-PE (eBioscience catalog number: 12-1079-42) and isotype control (eBioscience catalog number: 12-4714) antibodies were used to stain cell samples and were analyzed on a BD FACS Canto II flow cytometer. DNA dye SYTOX-Green (Life Technologies catalog number: S7020; 5 mM Solution in DMSO) was used. To achieve transfection efficiencies of up to 90% with high cell viability in KHYG-1 cells with the Nucleofector™ Device (Nucleofector II, Lonza), a Nucleofector™ Kit T from Lonza was used. Antibiotics-free RPMI 1640 containing 10% FBS, L-glutamine (2mM) and IL-2 (10ng/mL) was used for post-Nucleofection culture.

KHYG-1 and NK-92 cells were passaged one or two days before Nucleofection, as the cells must be in the logarithmic growth phase. The Nucleofector solution was pre-warmed to room temperature (100 μl per sample), along with an aliquot of culture medium containing serum and supplements at 37°C in a 50 mL tube. 6-well plates were prepared by filling with 1.5 mL culture medium containing serum and supplements and pre-incubated in a humidified 37°C / 5% $CO_2$ incubator. An aliquot of cell culture was prepared and the cells counted to determine the cell density. The required number of cells was centrifuged at 1500rpm for 5 min, before discarding the supernatant completely. The cell pellet was re-suspended in room temperature Nucleofector Solution to a final concentration of $2x10^6$ cells/100μl (maximum time in suspension = 20 minutes). 100 μl cell suspension was mixed with 10 μg mRNA (volume of RNA < 10 μL). The sample was transferred into an Amaxa-certified cuvette (making sure the sample covered the bottom of the cuvette and avoiding air bubbles). The appropriate Nucleofector program was selected (i.e. U-001 for KHYG-1 cells). The cuvettes were then inserted into the cuvette holder. 500 μl pre-warmed culture medium was added to the cuvette and the sample transferred into a prepared 6-well plate immediately after the program had finished, in order to avoid damage to the cells. The cells were incubated in a humidified 37°C / 5% $CO_2$ incubator. Flow cytometric analysis and cytotoxicity assays were performed 12-16 hours after electroporation. Flow cytometry staining was carried out as above.

Expression of TRAIL / TRAIL variant and CD107a (NK activation marker) increased post-transfection, confirming the successful knock-in of the TRAIL genes into KHYG-1 cells.

KHYG-1 cells were viable before and after transfection via electroporation. It was seen that no statistically significant differences in cell viability are observed following transfection of the cells with TRAIL / TRAIL variant, confirming that the expression of wildtype or variant TRAIL is not toxic to the cells. This observation contradicts corresponding findings in NK-92 cells, which suggest the TRAIL variant gene knock-in is toxic to the cells (data not shown). Nevertheless, this is likely explained by the relatively high expression levels of TRAIL receptors DR4 and DR5 on the NK-92 cell surface.

Effects of TRAIL / TRAIL variant on KHYG-1 Cell Cytotoxicity

Mouse anti-human CD2-APC antibody (BD Pharmingen catalog number: 560642) was used. Annexin V-FITC antibody (ImmunoTools catalog number: 31490013) was used. DNA dye SYTOX-Green (Life Technologies catalog number: S7020) was used. A 24-well cell culture plate (SARSTEDT AG catalog number: 83.3922) was used. Myelogenous leukemia cell line K562, multiple myeloma cell line RPMI8226 and MM1.S were used as target cells. K562, RPMI8226, MM1.S were cultured in RPMI 1640 medium containing 10% FBS, 2mM L-glutamine and penicillin (100 U/mL)/streptomycin (100 mg/mL).

As explained above, KHYG-1 cells were transfected with TRAIL / TRAIL variant.

The target cells were washed and pelleted via centrifugation at 1500rpm for 5 minutes. Transfected KHYG-1 cells were diluted to $0.5x10^6$/mL. The target cell density was then adjusted in pre-warmed RPMI 1640 medium, in order to produce effector:target (E:T) ratios of 1:1.

0.5 mL KHYG-1 cells and 0.5 mL target cells were then mixed in a 24-well culture plate and placed in a humidified 37°C / 5% $CO_2$ incubator for 12 hours. Flow cytometric analysis was then used to assay KHYG-1 cell cytotoxicity; co-cultured cells (at different time points) were washed and then stained with CD2-APC antibody (5 μL/test), Annexin V-FITC (5 μL/test) and SYTOX-Green (5 μL/test) using Annexin V binding buffer.

Data were further analyzed using FlowJo 7.6.2 software. CD2-positive and CD2-negative gates were set, which represent KHYG-1 cell and target cell populations, respectively. The Annexin V-FITC and SYTOX-Green positive cells in the CD2-negative population were then analyzed for TRAIL-induced apoptosis.

It is apparent for all target cell populations (K562, RPMI8226 and MM1.S) that TRAIL expression on KHYG-1 cells increased the level of apoptosis, when compared to normal KHYG-1 cells (not transfected with TRAIL). Moreover, TRAIL variant expression on KHYG-1 cells further increased apoptosis in all target cell lines, when compared to KHYG-1 cells transfected with wildtype TRAIL.

Cells of the invention, expressing the TRAIL variant, offer a significant advantage in cancer therapy, due to exhibiting higher affinities for the death receptor DR5. When challenged by these cells of the invention, cancer cells are prevented from developing defensive strategies to circumvent death via a certain pathway. Thus cancers cannot effectively circumvent TRAIL-induced cell death by upregulating TRAIL decoy receptors, as cells of the invention are modified so that they remain cytotoxic in those circumstances.

**Example 4 - Combined Expression of Mutant TRAIL Variant and Knockdown of CD96 in NK Cells**

Increases in NK cell cytotoxicity were observed when knocking down checkpoint inhibitory receptor CD96 expression and also when expressing TRAIL variant. We also tested combining the two genetic modifications to provoke a synergistic effect on NK cell cytotoxicity.

CD96 expression was knocked down in KHYG-1 cells, as described in Example 1.

KHYG-1 cells were transfected with the TRAIL variant (TRAIL D269H/E195R), as described above in Example 3.

(1) 12 hours after electroporation, KHYG-1 cells were co-cultured with target cells (K562 or MM1.S) at a concentration of $1x10^6$/mL in 12-well plates (2mL/well) for 12 hours. The E:T ratio was 1:1.
(2) 12 hours after co-culture, the cells were collected, washed, stained with CD2-APC, washed again and further stained with AnnexinV-FITC (5 uL/test) and SYTOX-Green (5 uL/test) using AnnexinV binding buffer.
(3) Cell samples were analyzed using a BD FACS canto II flow cytometer. Data were further analyzed using FlowJo 7.6.2 software. CD2-negative population represents MM1.S cells. KHYG-1 cells are strongly positive for CD2. The AnnexinV-FITC and SYTOX-Green positive cells in the CD2-negative population were then analyzed.

Simultaneously knocking down CD96 expression and expressing TRAIL variant in KHYG-1 cells was found to synergistically enhance the cells' cytotoxicity against both K562 target cells (Fig. 3) and MM1.S target cells (Fig. 4). This was indicated by the fact that in both target cell groups, more cell death resulted from the simultaneous genetic modification than resulted from the individual modifications in isolation.

At the same time, further evidence showing knockdown of CD96 increases NK cell cytotoxicity was obtained (Fig. 3 & 4), in addition to further evidence showing expression of the TRAIL mutant/variant increases NK cell cytotoxicity (Fig. 3 & 4).

**Example 5 - Knockdown of Checkpoint Inhibitory Receptor CD96 Function via CRISPR Gene Editing**

CRISPR gene editing was used to produce a KHYG-1 cell with a reduction in CD96 expression of 70% compared to wildtype CD96 expression in KHYG-1 cells.

A guide RNA was used to recognize the CD96 target site. The Cas9 nuclease was then used to bind to the specific site causing a double stranded break in the DNA. The CD96 gene attempts to repair itself, resulting in frameshift errors that prevent the transcription of the CD96 gene. As is known to the skilled person, RNA-guided Cas9 provides fast and easy implementation of RNA compared to other implementation systems, and also allows the production of stable cell lines.

The results of the CD96 knockdown using CRISPR can be seen in Figure 5: a histogram representation of CD96 expression following CRISPR knockdown compared to normal control KHYG-1 cells. The left-hand peak in each plot represents unstained samples and the right-hand peak represents stained samples. Normal control KHYG-1 had an MFI of 12,098, compared to CD96 knockdown cells with an MFI of 4,597. Analysis was done using FlowJo software.

As such, the cytokine profile of the CRISPR CD96 knockdown KHYG-1 cells was analyzed in the presence of multiple cancer cell lines.

**Example 6 - Cytokine Profile of NK Cells with Stable CD96 Knockdown**

It was found that KHYG-1 cells having CD96 knocked down via CRISPR exhibited improved cytotoxicity compared to wildtype KHYG-1 cells. Nevertheless, the CRISPR modified cells exhibited a slightly reduced increase in cytotoxicity when compared to those KHYG-1 cells having CD96 knocked down via RNAi (as seen in Examples 1, 2 and 4).

Specific cytotoxicity is, however, only one way by which an NK cell is capable of killing cancer cells *in vivo*. A more significant method is by way of recruiting other immune cells to the cancer microenvironment and mounting a full-scale immune response against the cancer. The way this is achieved is via cytokine signaling, specifically via IFNγ and TNFα.

To examine the release of cytokines within the co-culture model (n=3), five target cancer cell lines were used in combination with normal control KHYG-1 cells or CD96 KD KHYG-1 cells. Co-cultured was at a 1:1 E:T ratio for a total 24-hours. After incubation, the supernatants were collected and IFNγ and TNFα were analysed with an ELISA setup using a 1420 Multilabel counter plate reader. Readings were obtained at 405 and 595 nm. For both IFNγ and TNFα, a standard curve was obtained. Based on the standard curve, a protein concentration (pg) was calculated for each cell line.

IFNγ

Overall, IFNy production was significantly increased when CD96 knockdown KHYG-1 cells were co-cultured with myeloma cells, as compared to normal control KHYG-1 cells co-cultured with myeloma cells (see Figure 6). Significance was observed in 4 out of the 5 cancer cell lines tested. MM.1S showed an increase with p = 0.04, whereas both RPMI-8226 and U266 showed an increase with p = 0.03. In addition, H929 showed an increase with p=0.02. JJN3 was the only cancer cell line not to show a significant increase in IFNγ production. In the absence of cancer cells in the culture, there was no difference in cytokine production between the two KHYG-1 effector cell groups.

TNFα

Overall, TNFα production was significantly increased when CD96 knockdown KHYG-1 were co-cultured with myeloma cells, as compared to normal control KHYG-1 cells co-cultured with cancer cells (see Figure 7).. Significance was observed in 3 of the 5 cell lines. MM.1S showed an increase with p = 0.02, JJN3 showed an increase with p=0.04. In addition, H929 showed an increase with p=0.01. RPMI-8226 and U266 were the only cancer cell lines not to show a significant increase in TNFα production. In the absence of cancer cells in the culture, there was still an observed increase in cytokine production between the two KHYG-1 effector cell groups (p=0.02).

The two cell lines that showed significant increases in both TNFα and IFNγ were MM1.S and H929. It is of interest that both of these cell lines have the highest expression of CD111. CD111 has been associated with being an important predictor of the sensitivity of oncolytic therapy in several tumour types (Yu et al., 2007; Rueger et al., 2005; Huang et al., 2007). CD111 is considered a ligand for CD96, however this binding has previously only been observed in mice and not humans (Seth et al., 2007). Nevertheless, these data indicate a potential interplay between CD111 and CD96 in humans. As such, there is provided preliminary evidence for a new CD96 ligand in humans.

**Example 7 - Lentiviral Plasmids Encoding CD96-CARs**

DNA nucleotides encoding a single chain variable fragment (scFv) that recognises CD96 (SEQ ID NO: 1), were cloned into pCDCAR1-GFP vectors. This scFv sequence was followed by an additional immunoglobulin-based hinge region to overcome any stearic hindrance of CD96. The hinge regions were followed by the co-stimulatory activating domains of CD28, OX40 and CD3zeta, in order to provide activating signals for NK cell cytotoxicity, thereby triggering cytolysis of CD96 positive tumour cells. Following the chimeric antigen receptor (CAR) protein (SEQ ID NO: 2), the selection marker Enhanced Green Fluorescent Protein (EGFP) was positioned downstream of the T2A sequence, separating the CAR and the EGFP. The sequence was cloned between EcoRI and Xbal restriction sites of the pCDCAR1-GFP vector.

150μl vials of competent E. coli bacteria were thawed on ice. In the meantime, unlabelled tubes were chilled on ice. Once the last crystal had melted, 50μl of the cells were promptly transferred to each chilled tube, along with 1μl of the appropriate diluted plasmid solution (5ng/μl). The tubes were then left on ice for 30 minutes. The cells were heat-shocked by submerging the tubes in a water bath set at 42°C for 30 seconds and then allowing them to cool back on the ice for 5 minutes. 950μl of room temperature LB broth was added to each tube and then the tubes were flicked gently to make a uniform mixture. The tubes were placed on a shaker (250rpm) in the incubator (37°C) for 1 hour. Next, 50μl of each plasmid solution was added into its corresponding agar plate containing ampicillin. The plates were stored in the incubator upside down overnight to allow bacteria containing the plasmid to grow out and form single colonies (14-16hrs). A colony was selected from one of the plates. Using an autoclaved pipette tip, a colony was taken up and smeared against the inner surface of the corresponding culture tube. The tubes were then left on a shaker (125rpm) in the incubator (37°C) overnight for a maximum of 16 hours. The plasmid DNA was isolated according to the manufacturer's instructions. The plasmid was verified by restriction digestion with restriction enzymes. The bacterial culture (2.5ml) was added to 247.5ml LB broth containing Ampicillin. The 250ml bacterial solution was then split into 50ml tubes and stored at -20°C.

The labelled 50ml tubes containing the bacterial cell pellets were removed from the - 20°C freezer and allowed to thaw on the bench for a few minutes. The first cell pellet was vortexed and re-suspended in 12ml of re-suspension buffer containing RNase A. 12ml of Lysis Buffer was added to the suspensions. The suspensions were inverted for mixing and then incubated at room temperature for 5 minutes. 12ml of neutralization buffer was added to each suspension, followed by inverting the tubes until the sample turned from blue to colourless. The crude lysates were then incubated on ice for 5 minutes. A column filter was inserted into NucleoBond Xtra columns, before applying 15ml of Equilibration buffer onto the rim of each column filter to ensure moistening of the entire column and its circumference. The column was emptied by gravity flow into a basin under a column rack. The lysates were poured into their designated column filters. The column filters were washed with 5ml Filter Wash Buffer, making sure to apply the buffer to the funnel shaped rim of the filter as mentioned above. The column was then emptied by gravity flow. The column filter was removed and discarded. The NucleoBond Xtra Column was washed with 35ml of Wash Buffer, before allowing the column to empty by gravity flow. For washes of the column, the buffer was gently pipetted directly into the centre as opposed to on the rim. This wash step was then repeated. A 15ml tube was positioned underneath each of the columns to collect elute. The plasmid DNA was eluted from the columns with 5ml of Elution Buffer. 3.5ml of room-temperature isopropanol was pipetted into each 15ml tube, in order to precipitate the eluted plasmid DNA. The tubes were then vortexed thoroughly. The tubes were centrifuged at 4500rpm for 40 minutes at 4°C, before carefully aspirating the supernatants. 2ml of endotoxin-free room-temperature 70% ethanol was added to each pellet. The tubes were then centrifuged at 4500 x g for 10 minutes at room temperature. The ethanol from each tube was removed using a pipette and left to dry under a hood at room temperature until the pellets turned from being slightly opaque to a more translucent glassy appearance. The DNA concentration was then measured using a Nanodrop spectrophotometer, and the DNA pellets were dissolved in 400μl of H2O-EF.

The sequence of the CD96-scFv and activating domains of the CD96-CAR are as follows:

| Sequence position | Sequence Type | Base Pair length |
|---|---|---|
| 1 | Signal peptide | 48 bp |
| 2 | VL (Variable region light chain) | 354 bp |
| 3 | (G4S)3 Linker sequence | 60 bp |
| 4 | VH (Variable region heavy chain) | 348 bp |
| 5 | IgD Hinge | 309 bp |
| 6 | IgG1 Fc+ Hinge | 699 bp |
| 7 | CD28 | 204 bp |
| 8 | OX40 | 111 bp |
| 9 | CD3zeta | 336 bp |

The final CAR construct comprised the co-stimulatory domain CD28 in combination with the signaling domain CD3zeta, as found to be optimal in NK cell systems.

**Example 8 - Nucleofection of CD96-CAR Plasmids into KHYG-1 Cells**

KHYG-1 cells were passaged at 1:1 (5ml cells + 5ml culture media) the day before nucleofection in T25 flasks, while the cells were in the logarithmic growth phase. The Lonza Nucleofection kit T (Cat: VCA-1002) was used; one nucleofection sample contained 100μl nucleofection solution (standard cuvette) and $2x10^6$ cells. The nucleofection solution contained 18μl Supplement and 82μl Nulceofector solution (per sample) prepared fresh and incubated at 37°C for 10 minutes.

Solution T was warmed to room temperature. A fresh 12ml aliquot of culture medium (CM) was prepared containing 2.4ml FBS, 9.6ml RPMI 1640 and supplements 6μl IL-2 (RPMI1640+ 20% FBS + 500 IU/ml IL-2) at 37°C in a 15ml tube (no antibiotics). 4ml of CM was aliquoted into T25 flasks, and plates were pre-incubated in a humidified 37°C incubator for 20 minutes. 10ml cell culture in 15ml tubes was taken and the cells were counted to determine the cell density. A $2x10^6$ sample was dispensed into 15 ml tubes and centrifuged at 1000 RPM for 5 min (acc. 9/decc.7). The supernatant completely discarded so that no residual medium covered the cell pellet. The cell pellet was re-suspended in 100μl room temperature Nucleofector Solution (see above) to a final concentration of $2x10^6$ cells/100μl. Storing the cell suspension longer than 5min in Nucleofector Solution was avoided, as this is known to reduce cell viability and gene transfer efficiency. 02-2μg Lenti-EF1a-CD96-CAR Plasmid DNA was added to each tube. The sample was then transferred into an Amaxa certified cuvette, making sure that the sample covered the bottom of the cuvette, in order to avoid air bubbles while pipetting. The cuvette was closed with a blue cap and Nucleofector program A-024 was selected. The cuvette was inserted into the cuvette holder, before pressing the "x" button to start the program (NB: program U-001 can also be used). To avoid damage to the cells, the samples were removed from the cuvette immediately after the program had finished (display showing "OK"). The pre-warmed culture medium was added to the cuvette and the sample transferred into one T25 flask. The "X" button was pressed to reset the Nucleofector. The cells were incubated in a humidified 37°C / 5% $CO_2$ incubator for 48 hours. 2-4ml fresh media (RPM11640 + 10% FBS + 100 IU/ml IL-2) was added and the cells were incubated for another 24-96 hours to establish optimal protein expression (NB: the cell culture was monitored after every 24 hours using a microscope to check the status and condition of the cells). Flow cytometric analysis was performed between 72 and 240 hours post-nucleofection.

The CD96 CAR plasmid had EGFP as a selection marker. Therefore, KHYG-1 cells expressing CD96 CARs were determined by estimating the number of GFP-positive KHYG-1 cells.

0.5ml cell suspension ($1x10^5$ cells) was centrifuged at 1200rpm for 5 mins. The supernatant was discarded, before adding 1ml FACS buffer and centrifuging again at 1200rpm for 5 mins. The cells were re-suspended in a final volume of 200μl FACS buffer.

The cells were then analysed by flow cytometry using a 488nm laser and detection filter of 530/30 on a FACS Canto A - presence of GFP-positive cells confirmed successful transfection.

The NK cells of the invention are modified to express CD96 CARs and have CD96 expression removed. As such, the cells of the invention are of particular use in the treatment of CD96-expressing cancers.

The invention thus provides NK cells and cell lines, and production thereof, for use in cancer therapy.

## Claims

1. A natural killer (NK) cell or NK cell line, wherein the NK cell or NK cell line expresses a ligand that binds CD96 and wherein the NK cell or NK cell line is not itself bound by CD96 ligand.

2. An NK cell or NK cell line according to claim 1, wherein the NK cell or NK cell line has been modified to express a chimeric antigen receptor (CAR) for CD96.

3. An NK cell or NK cell line according to any preceding claim, wherein the NK cell or NK cell line has been modified to express a natural ligand for CD96.

4. An NK cell or NK cell line according to claim 3, wherein the natural CD96 ligand is CD155.

5. An NK cell or NK cell line according to claim 3, wherein the natural CD96 ligand, e.g. CD155, is modified to have a higher affinity for CD96 than the wildtype ligand.

6. An NK cell or NK cell line according to any preceding claim, wherein the NK cell or NK cell line does not itself express CD96.

7. An NK cell or NK cell line according to any preceding claim, wherein CD96 expression in the NK cell or NK cell line is knocked out or knocked down, e.g. CD96 expression is reduced by at least 80% compared to the same NK cell or NK cell line without CD96 being knocked down.

8. An NK cell or NK cell line according to any preceding claim, wherein the NK cell or NK cell line has been modified to express CD96 and/or TIGIT linked to a co-stimulatory domain, so as to convert CD96 and/or TIGIT into an activating receptor.

9. An NK cell or NK cell line according to claim 8, wherein the co-stimulatory domain is selected from 41BB (CD137), CD28, OX40, CD3zeta (CD247), DAP12 and/or DAP10.

10. An NK cell or NK cell line according to any preceding claim, wherein the NK cell or NK cell line has been modified to have absent or reduced expression of the checkpoint inhibitory receptor TIGIT.

11. An NK cell or NK cell line according any preceding claim, wherein the NK cell or NK cell line has been modified to express a mutant form of TRAIL ligand, wherein the mutant TRAIL ligand has an increased affinity for one or more TRAIL death receptors, e.g. DR4 and/or DR5.

12. An NK cell or NK cell line according any preceding claim, for use in treating cancer.

13. An NK cell or NK cell line for use according claim 12, wherein the cancer is a blood cancer.

14. An NK cell or NK cell line for use according claim 13, wherein the blood cancer is selected from acute lymphocytic leukemia (ALL), T-cell acute lymphoblastic leukemia (T-ALL) and acute myeloid leukemia (AML).

15. An NK cell or NK cell line for use according to claim 12, wherein the stem cell cancer derives from leukemic stem cells.

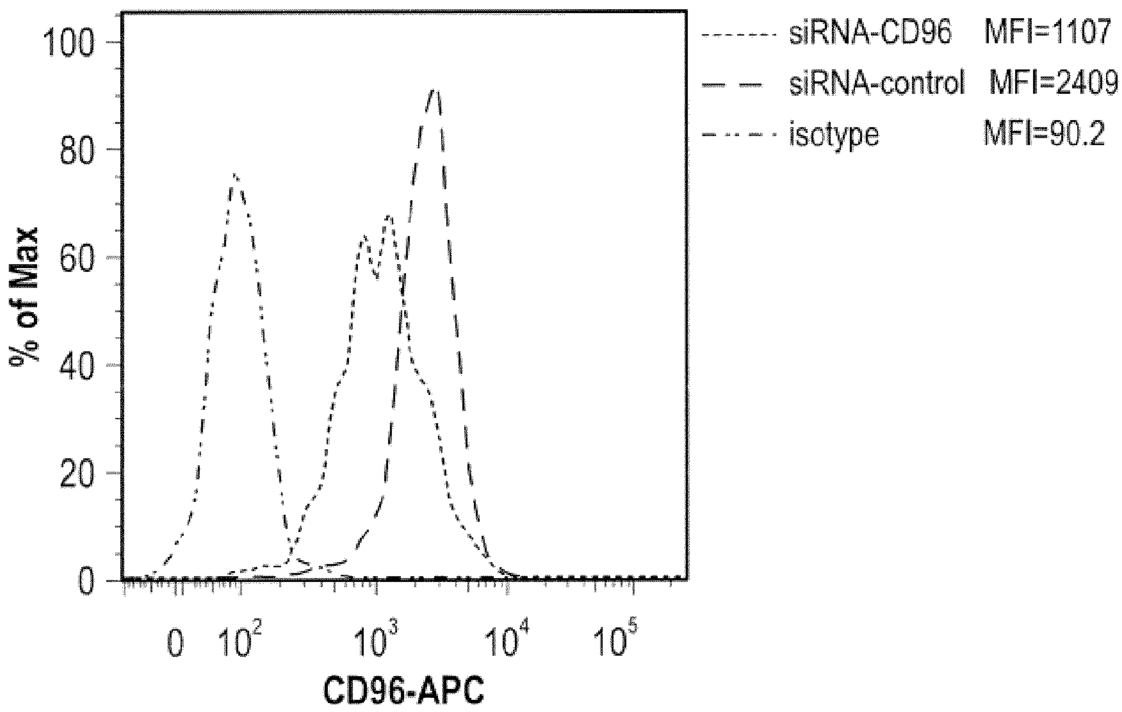
siRNA-CD96 MFI=1107
siRNA-control MFI=2409
isotype MFI=90.2
% of Max
100
80
60
40
20
0
0 10^2 10^3 10^4 10^5
CD96-APC

Fig. 1A

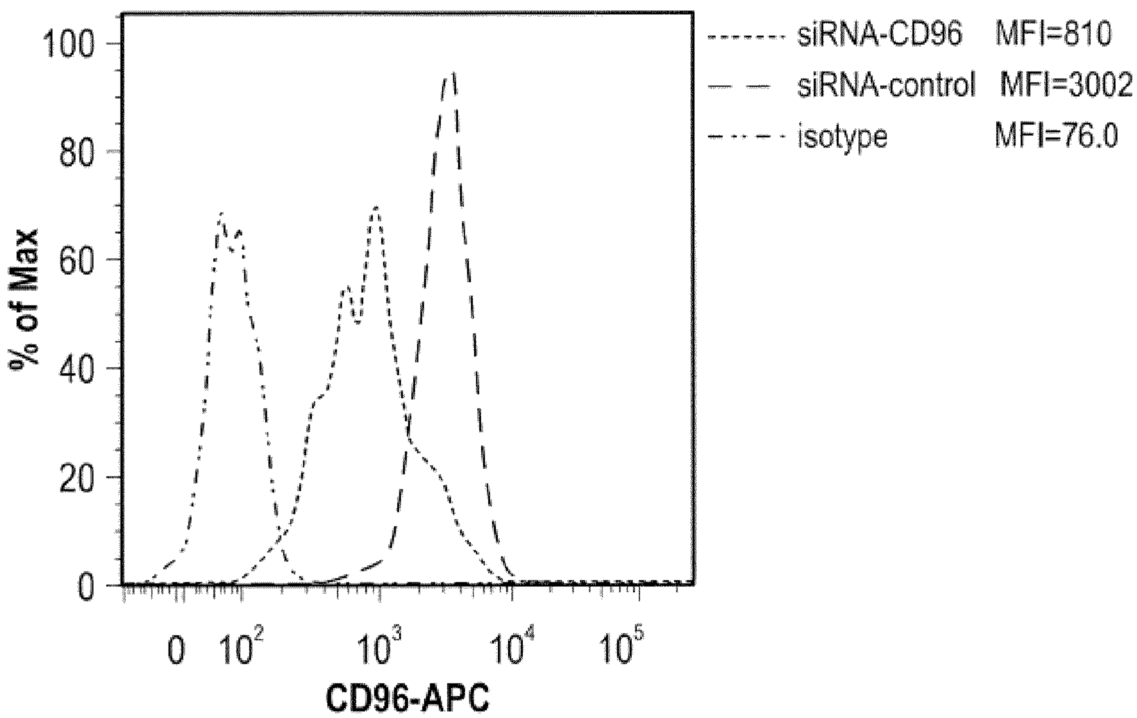
siRNA-CD96 MFI=810
siRNA-control MFI=3002
isotype MFI=76.0
% of Max
100
80
60
40
20
0
0 10^2 10^3 10^4 10^5
CD96-APC

Fig. 1B

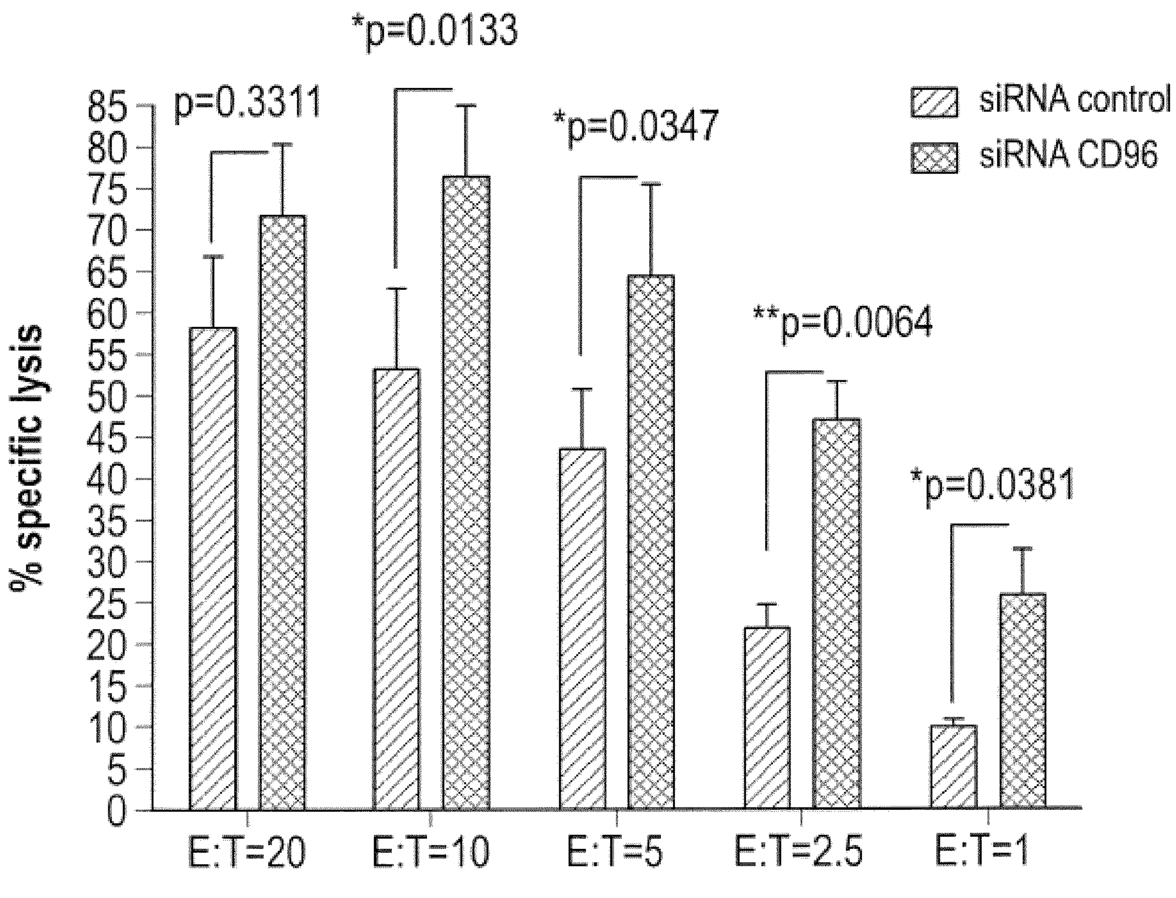
*p=0.0133
p=0.3311
*p=0.0347
**p=0.0064
*p=0.0381
siRNA control
siRNA CD96
% specific lysis
0
5
10
15
20
25
30
35
40
45
50
55
60
65
70
75
80
85
E:T=20
E:T=10
E:T=5
E:T=2.5
E:T=1

**Fig. 2**

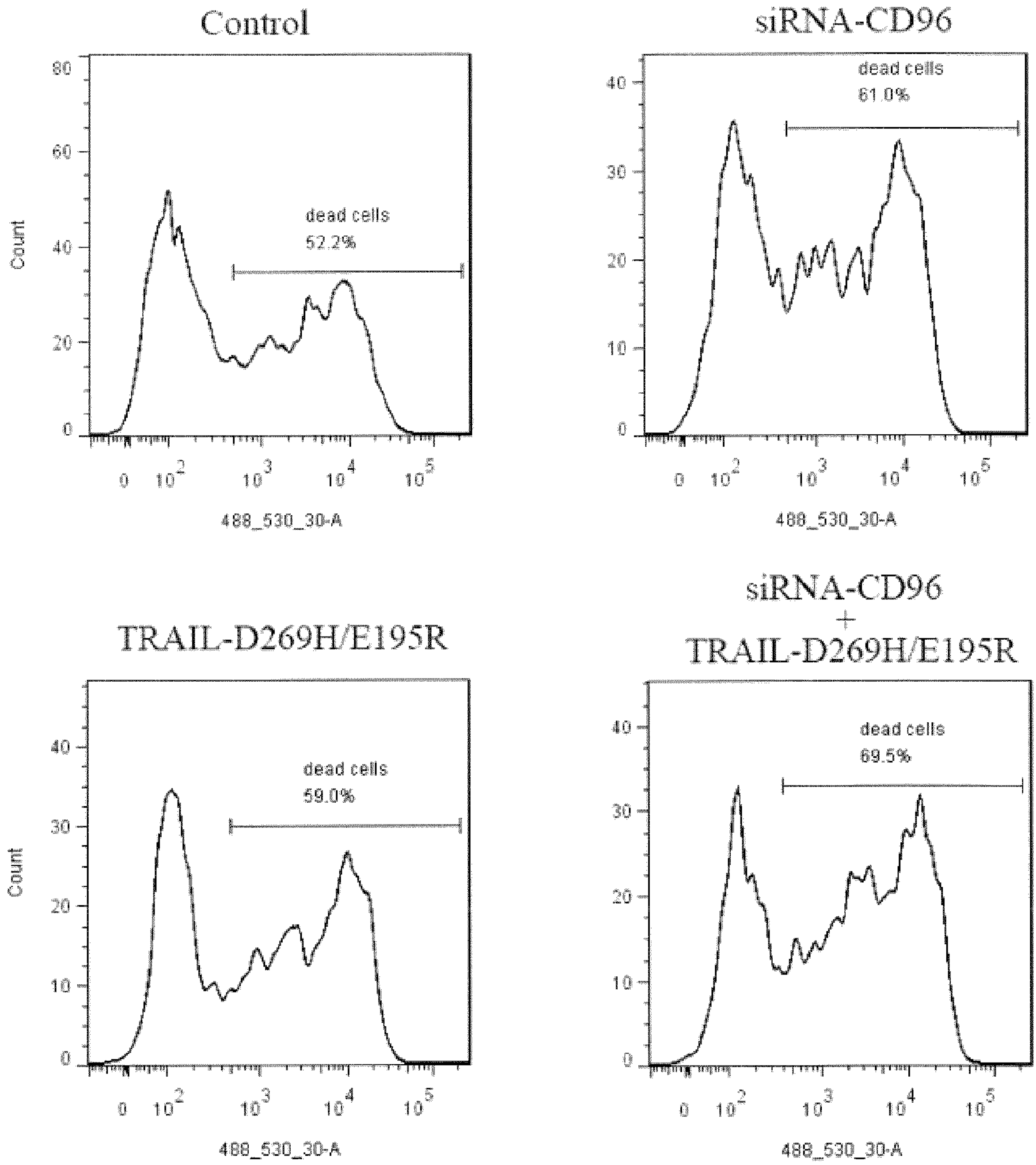
Control
Count
dead cells
52.2%
488_530_30-A
siRNA-CD96
dead cells
61.0%
488_530_30-A
TRAIL-D269H/E195R
Count
dead cells
59.0%
488_530_30-A
siRNA-CD96
+
TRAIL-D269H/E195R
dead cells
69.5%
488_530_30-A

**Fig. 3**

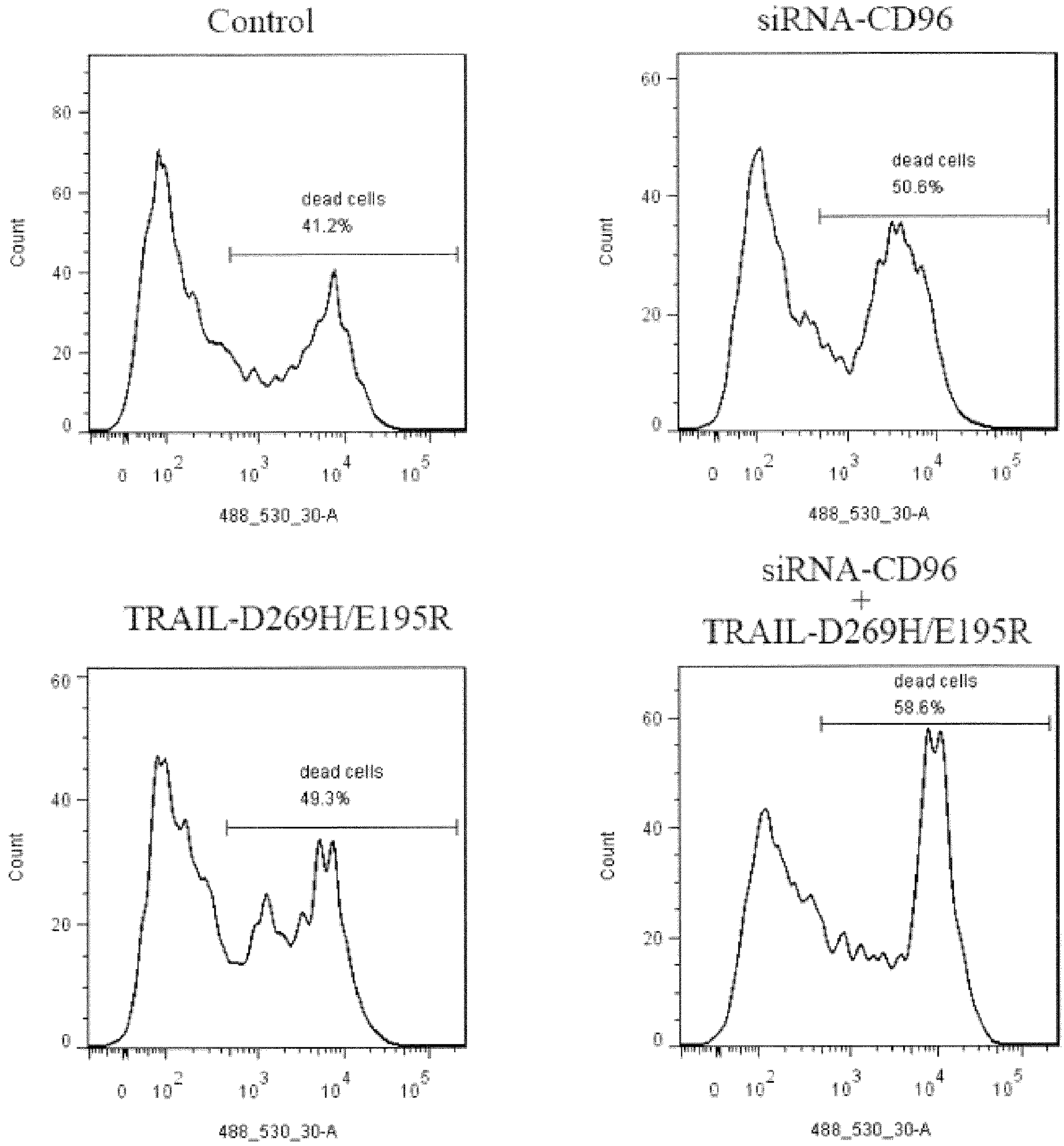
Control
dead cells
41.2%
Count
488_530_30-A
siRNA-CD96
dead cells
50.6%
Count
488_530_30-A
TRAIL-D269H/E195R
dead cells
49.3%
Count
488_530_30-A
siRNA-CD96
+
TRAIL-D269H/E195R
dead cells
58.6%
Count
488_530_30-A

**Fig. 4**

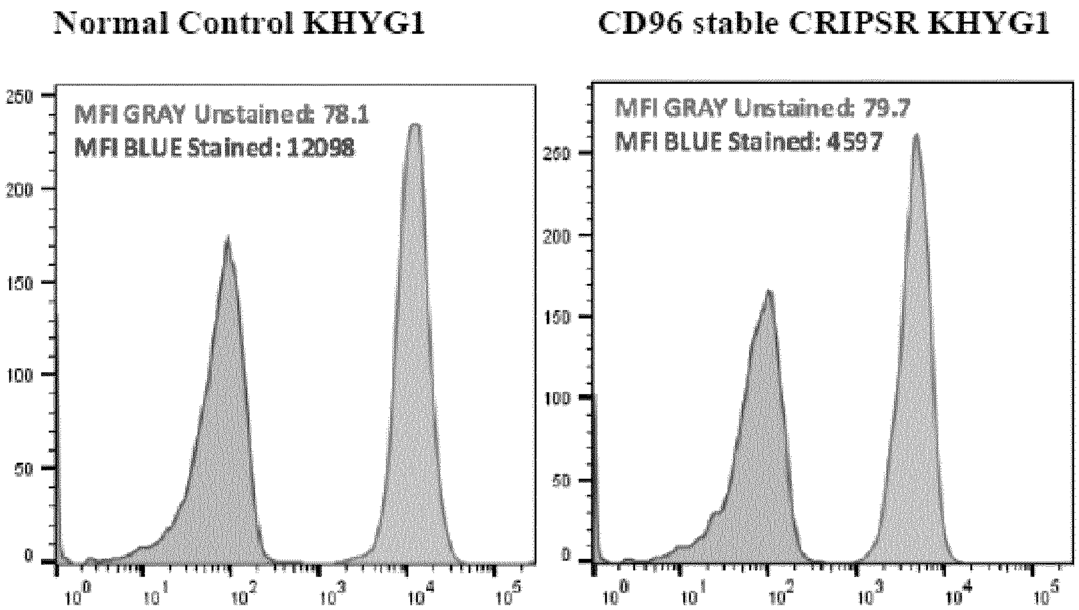
Normal Control KHYG1
CD96 stable CRIPSR KHYG1
MFI GRAY Unstained: 78.1
MFI BLUE Stained: 12098
MFI GRAY Unstained: 79.7
MFI BLUE Stained: 4597

Fig. 5

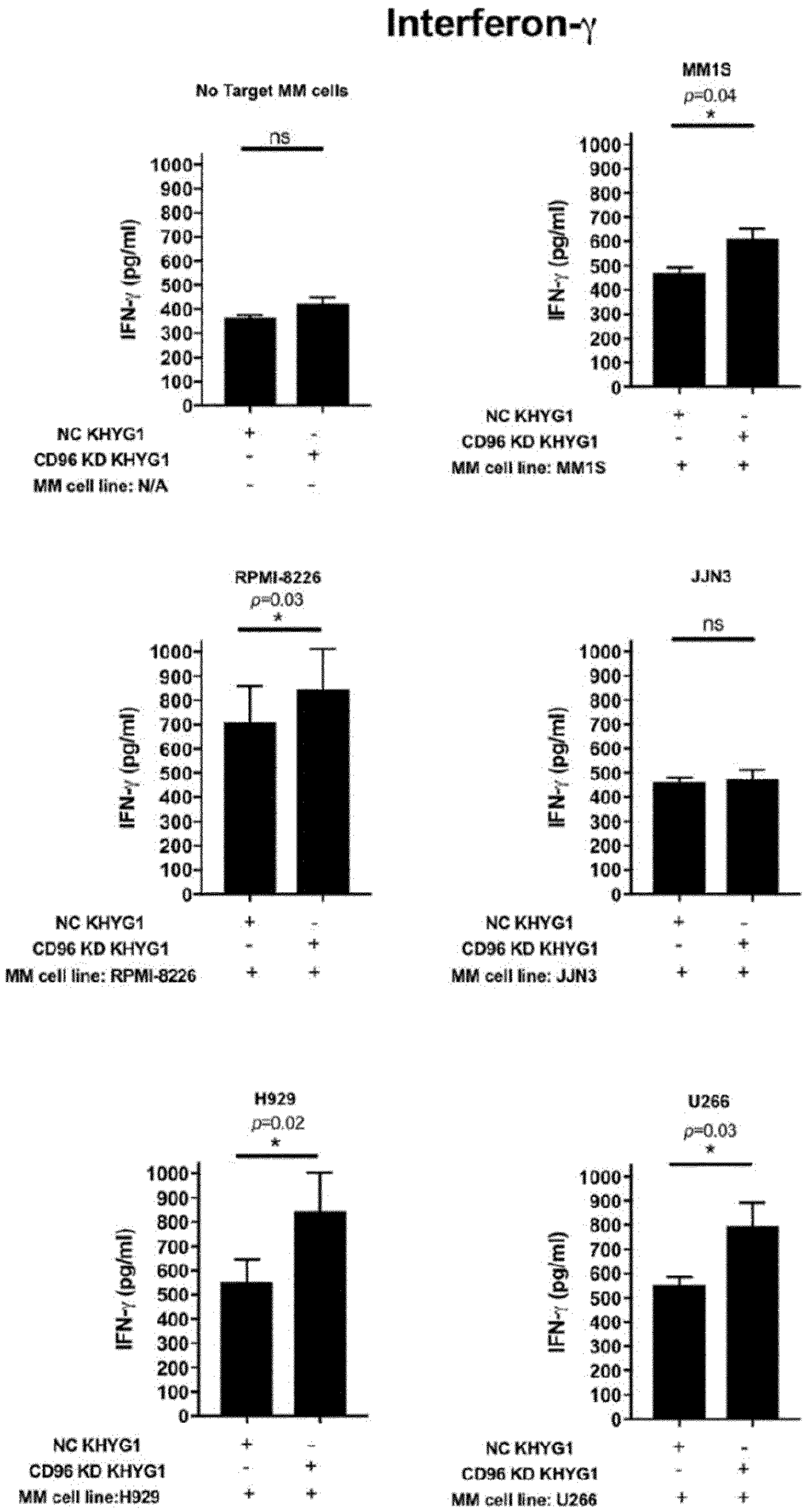
Interferon-γ
No Target MM cells
ns
IFN-γ (pg/ml)
NC KHYG1
CD96 KD KHYG1
MM cell line: N/A
MM1S
p=0.04
*
MM cell line: MM1S
RPMI-8226
p=0.03
MM cell line: RPMI-8226
JJN3
ns
MM cell line: JJN3
H929
p=0.02
MM cell line:H929
U266
p=0.03
MM cell line: U266

Fig. 6

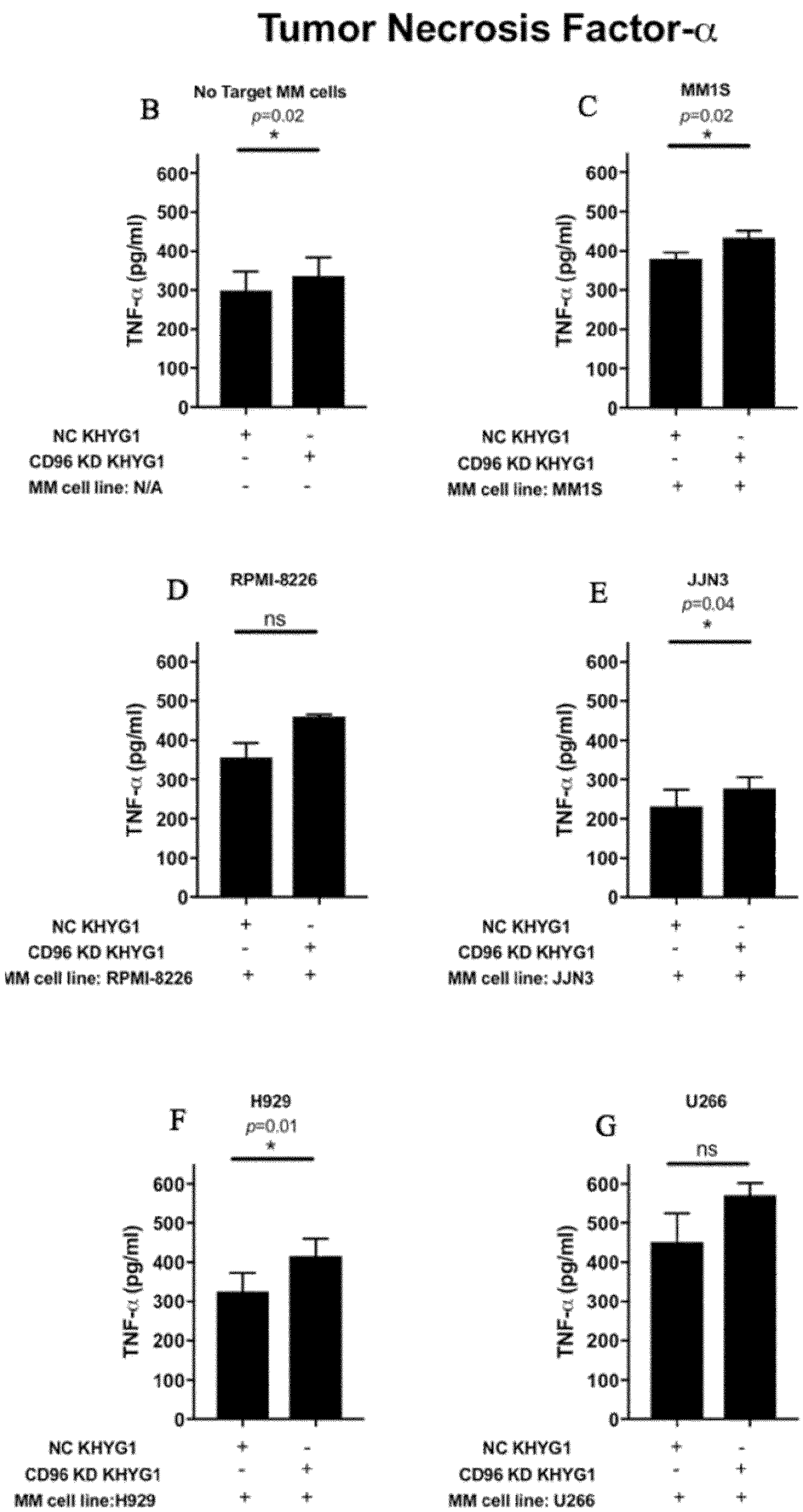
Tumor Necrosis Factor-α
B
No Target MM cells
p=0.02
*
TNF-α (pg/ml)
600
500
400
300
200
100
0
NC KHYG1 + -
CD96 KD KHYG1 - +
MM cell line: N/A - -
C
MM1S
p=0.02
*
TNF-α (pg/ml)
NC KHYG1 + -
CD96 KD KHYG1 - +
MM cell line: MM1S + +
D
RPMI-8226
ns
TNF-α (pg/ml)
NC KHYG1 + -
CD96 KD KHYG1 - +
MM cell line: RPMI-8226 + +
E
JJN3
p=0.04
*
TNF-α (pg/ml)
NC KHYG1 + -
CD96 KD KHYG1 - +
MM cell line: JJN3 + +
F
H929
p=0.01
*
TNF-α (pg/ml)
NC KHYG1 + -
CD96 KD KHYG1 - +
MM cell line:H929 + +
G
U266
ns
TNF-α (pg/ml)
NC KHYG1 + -
CD96 KD KHYG1 - +
MM cell line: U266 + +

**Fig. 7**

European Patent Office / Europäisches Patentamt / Office européen des brevets

# EUROPEAN SEARCH REPORT

Application Number
EP 19 19 5985

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2017/017184 A1 (ONKIMMUNE LTD [IE]) 2 February 2017 (2017-02-02)<br>* claims 1-20; examples 2-3 * | 1<br>2-15 | INV.<br>C12N5/0783<br>A61K35/17<br>A61K38/17<br><br>ADD.<br>C07K14/47<br>C07K14/705 |
| X<br>Y | US 2019/175654 A1 (SWANSON RYAN [US] ET AL) 13 June 2019 (2019-06-13)<br>* paragraph [0003] *<br>* paragraph [0012] *<br>* paragraph [0090] * | 1<br>2-15 | |
| A | WO 2017/021526 A1 (AMGEN RES GMBH [DE]) 9 February 2017 (2017-02-09)<br>* claims 1-28 * | 1-15 | |
| Y | WO 2018/129346 A1 (NANTKWEST INC [US]) 12 July 2018 (2018-07-12)<br>* paragraph [0006] *<br>* paragraph [0007] *<br>* paragraph [0032] *<br>* paragraph [0161] * | 2-15 | |
| A | KARADE SHARANBASAPPA S ET AL: "How Natural Killer Cell Receptors Stick to Cell-Cell Adhesion Proteins", STRUCTURE, vol. 27, no. 2, 5 February 2019 (2019-02-05), pages 209-210, XP085591883, ISSN: 0969-2126, DOI: 10.1016/J.STR.2019.01.007<br>* the whole document * | 1-15 | TECHNICAL FIELDS SEARCHED (IPC)<br>C12N<br>A61K<br>C07K |

-/--

The present search report has been drawn up for all claims

| Place of search | Date of completion of the search | Examiner |
|---|---|---|
| Munich | 20 February 2020 | Paresce, Donata |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 1 of 3

Europäisches Patentamt
European Patent Office
Office européen des brevets

# EUROPEAN SEARCH REPORT

Application Number
EP 19 19 5985

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | HRISTO GEORGIEV ET AL: "Coming of Age: CD96 Emerges as Modulator of Immune Responses", FRONTIERS IN IMMUNOLOGY, vol. 9, 17 May 2018 (2018-05-17), XP055670063, DOI: 10.3389/fimmu.2018.01072 * the whole document * ----- | 1-15 | |
| A | KUCAN BRLIC PAOLA ET AL: "Targeting PVR (CD155) and its receptors in anti-tumor therapy", CELLULAR & MOLECULAR IMMUNOLOGY, CHINESE SOCIETY OF IMMUNOLOGY, CH, vol. 16, no. 1, 1 October 2018 (2018-10-01), pages 40-52, XP036683851, ISSN: 1672-7681, DOI: 10.1038/S41423-018-0168-Y [retrieved on 2018-10-01] * the whole document * ----- | 1-15 | TECHNICAL FIELDS SEARCHED (IPC) |
| A | CHRISTOPHER J CHAN ET AL: "The receptors CD96 and CD226 oppose each other in the regulation of natural killer cell functions", NATURE IMMUNOLOGY, vol. 15, no. 5, 23 March 2014 (2014-03-23), pages 431-438, XP055344476, New York ISSN: 1529-2908, DOI: 10.1038/ni.2850 * the whole document * ----- -/-- | 1-15 | |

The present search report has been drawn up for all claims

| Place of search | Date of completion of the search | Examiner |
|---|---|---|
| Munich | 20 February 2020 | Paresce, Donata |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 3

Europäisches Patentamt
European Patent Office
Office européen des brevets

# EUROPEAN SEARCH REPORT

Application Number
EP 19 19 5985

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | S. J. BLAKE ET AL: "Molecular Pathways: Targeting CD96 and TIGIT for Cancer Immunotherapy", CLINICAL CANCER RESEARCH, vol. 22, no. 21, 1 November 2016 (2016-11-01), pages 5183-5188, XP055419815, US ISSN: 1078-0432, DOI: 10.1158/1078-0432.CCR-16-0933 * the whole document * ----- | 1-15 | |

TECHNICAL FIELDS SEARCHED (IPC)

The present search report has been drawn up for all claims

| Place of search | Date of completion of the search | Examiner |
|---|---|---|
| Munich | 20 February 2020 | Paresce, Donata |

2

EPO FORM 1503 03.82 (P04C01)

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

page 3 of 3

## ANNEX TO THE EUROPEAN SEARCH REPORT ON EUROPEAN PATENT APPLICATION NO. EP 19 19 5985

This annex lists the patent family members relating to the patent documents cited in the above-mentioned European search report. The members are as contained in the European Patent Office EDP file on
The European Patent Office is in no way liable for these particulars which are merely given for the purpose of information.

20-02-2020

| Patent document cited in search report | Publication date | Patent family member(s) | Publication date |
|---|---|---|---|
| WO 2017017184 A1 | 02-02-2017 | AU 2016300128 A1 | 22-02-2018 |
| | | BR 112018001660 A2 | 18-09-2018 |
| | | CA 2993796 A1 | 02-02-2017 |
| | | CN 108026512 A | 11-05-2018 |
| | | DK 3317401 T3 | 02-01-2019 |
| | | EP 3317401 A1 | 09-05-2018 |
| | | EP 3421590 A1 | 02-01-2019 |
| | | EP 3434762 A1 | 30-01-2019 |
| | | ES 2701338 T3 | 21-02-2019 |
| | | HK 1252582 A1 | 31-05-2019 |
| | | JP 2018522592 A | 16-08-2018 |
| | | KR 20180028530 A | 16-03-2018 |
| | | RU 2018104871 A | 28-08-2019 |
| | | US 2017157230 A1 | 08-06-2017 |
| | | US 2018326029 A1 | 15-11-2018 |
| | | WO 2017017184 A1 | 02-02-2017 |
| US 2019175654 A1 | 13-06-2019 | CA 3032120 A1 | 01-02-2018 |
| | | CN 110088127 A | 02-08-2019 |
| | | EP 3491013 A1 | 05-06-2019 |
| | | US 2019175654 A1 | 13-06-2019 |
| | | WO 2018022946 A1 | 01-02-2018 |
| WO 2017021526 A1 | 09-02-2017 | AU 2016301961 A1 | 18-01-2018 |
| | | CA 2996015 A1 | 09-02-2017 |
| | | CN 108473572 A | 31-08-2018 |
| | | EA 201890428 A1 | 31-07-2018 |
| | | EP 3331915 A1 | 13-06-2018 |
| | | JP 2018531215 A | 25-10-2018 |
| | | PH 12018500120 A1 | 23-07-2018 |
| | | TW 201718002 A | 01-06-2017 |
| | | US 2017037133 A1 | 09-02-2017 |
| | | US 2019077869 A1 | 14-03-2019 |
| | | WO 2017021526 A1 | 09-02-2017 |
| WO 2018129346 A1 | 12-07-2018 | AU 2018206401 A1 | 04-07-2019 |
| | | CA 3048907 A1 | 12-07-2018 |
| | | CN 110168078 A | 23-08-2019 |
| | | EP 3565889 A1 | 13-11-2019 |
| | | KR 20190095959 A | 16-08-2019 |
| | | WO 2018129346 A1 | 12-07-2018 |



For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2009077857 A

SEQUENCE LISTING

<110> ONK Therapeutics Limited

<120> CELLULAR THERAPIES FOR CANCER

<130> P40561EP

<160> 2

<170> PatentIn version 3.5

<210> 1
<211> 2469
<212> DNA
<213> Human

<220>
<221> misc_feature
<222> (1)..(48)
<223> Signal Peptide

<220>
<221> misc_feature
<222> (49)..(402)
<223> Variable Region Light Chain

<220>
<221> misc_feature
<222> (403)..(462)
<223> (G4S)3 Linker

<220>
<221> misc_feature
<222> (463)..(810)
<223> Variable Region Heavy Chain

<220>
<221> misc_feature
<222> (811)..(1119)
<223> IgD Hinge

<220>
<221> misc_feature
<222> (1120)..(1818)
<223> IgG1 Fc+ Hinge

<220>
<221> misc_feature
<222> (1819)..(2022)
<223> CD28

<220>
<221> misc_feature
<222> (2023)..(2133)
<223> OX40

<220>
<221> misc_feature
<222> (2134)..(2469)
<223> CD3zeta

<400> 1
atgtggcaac tgctgctgcc tacagctctg ctgcttctgg tgtccgccgc catggcggac 60

tacaaagaca ttgtgctgac ccaatctcca gcttctttgg ctgtgtctct agggcagagg 120

gccaccatat cctgcagagc cagtgaaagt gttgatagtt atggcaatag ttttatgcac 180

tggtaccagc agaaaccagg acagccaccc aaactcctca tctatcgtgc atccaattta 240

gaatctggga tccctgccag gttcagtggc agtgggtcta ggacagactt caccctcacc 300

attaatcctg tggaggctga tgatgttgca acctattact gtcagcaaag taatgaggat 360

ccgtacacgt tcggaggggg gaccaagctg gaaataaaac gtggtggtgg tggttctggt 420

ggtggtggtt ctggcggcgg cggctccggt ggtggtggat ccgaggtgca gctgaaggag 480

tctggccctg ggatattgca gccctcccag accctcagtc tgacttgttc cttctctggg 540

ttttcactga acacctctgg tatgggtgta ggctggattc gtcagccttc agggaagggt 600

ctggagtggc tggcacacat ttggtgggat gatgacaagc gctataaccc agccctgaag 660

agccgactga cattctccaa ggatacctcc agcaaccagg tattcctcaa gatcgccagt 720

gtggacactg cagatactgc cacatactac tgtgttcgag gggtaactc gtttgcttac 780

tggggccaag ggactctggt cactgtctct accttcacct gttttgtcgt gggcagcgac 840

ctgaaggatg cccacctgac atgggaagtc gccggcaaag ttcctaccgg tggcgtggaa 900

gaaggcctgc tggaaagaca cagcaacggc agccagagcc agcacagcag actgacactg 960

cctagaagcc tgtggaatgc cggcaccagc gtgacctgca cactgaatca tcctagcctg 1020

cctccacaga gactgatggc cctgagagaa cctgctgctc aggcccctgt gaagctgtcc 1080

ctgaatctgc tcgccagcag cgatcctcct gaagccgcca atgtgaacca caagcctagc 1140

aacaccaagg tggacaagaa ggtggaaccc aagagctgcg acaagaccca cacctgtcct 1200

ccatgtcctg ctccagaact gctcggcgga ccttccgtgt tcctgtttcc tccaaagcct 1260

aaggacaccc tgatgatcag cagaacccct gaagtgacct gcgtggtggt ggatgtgtcc 1320

cacgaggatc ccgaagtgaa gttcaattgg tacgtggacg gcgtcgaggt gcacaacgcc 1380

aagacaaagc ccagagagga acagtacaac agcacctaca gagtggtgtc cgtgctgacc 1440

gtgctgcacc aggattggct gaacggcaaa gagtacaagt gcaaggtgtc caacaaggcc 1500

ctgcctgctc ctatcgagaa aaccatcagc aaggccaagg gccagcctag agaaccccag 1560

gtgtacacac tgcctccaag cagagatgag ctgaccaaga accaggtgtc cctgacatgc 1620

ctggtcaagg gcttctaccc ctccgatatc gccgtggaat gggagagcaa tggacagccc 1680

gagaacaact acaagacaac ccctcctgtg ctggactccg acggctcatt cttcctgtac 1740

agcaaactga ccgtggacaa gtccagatgg cagcagggca acgtgttctc ctgcagcgtg 1800

atgcacgagg ccctgcactt ttgggtgctc gtggttgttg gcggagtgct ggcctgttac 1860

agcctgctgg ttaccgtggc cttcatcatc ttttgggtcc gaagcaagcg gagccggctg 1920

ctgcacagcg actacatgaa catgacccct agacggcccg gacctaccag aaagcactac 1980

cagccttacg ctcctcctag agacttcgcc gcctacagaa gcagacggga ccaaagactg 2040

cctcctgacg ctcacaaacc tccaggcggc ggaagcttca ggacccctat ccaagaagaa 2100

caggctgacg cccacagcac cctggccaag atccgcgtga agttctccag atccgccgac 2160

gctcctgcct atcagcaggg acagaaccag ctgtacaacg agctgaacct ggggagaaga 2220

gaagagtacg acgtgctgga taagcggaga ggcagagatc ctgagatggg cggaaagccc 2280

cagcggagaa agaatcctca agagggcctg tataatgagc tgcagaaaga caagatggcc 2340

gaggcctaca gcgagatcgg aatgaagggc gagcgcagaa gaggcaaggg acacgatgga 2400

ctgtaccagg gactgagcac cgccaccaag gatacctatg acgccctgca catgcaggct 2460

ctgcctcca 2469

<210> 2
<211> 823
<212> PRT
<213> Human

<400> 2

Met Trp Gln Leu Leu Leu Pro Thr Ala Leu Leu Leu Leu Val Ser Ala
1 5 10 15

Ala Met Ala Asp Tyr Lys Asp Ile Val Leu Thr Gln Ser Pro Ala Ser
20 25 30

Leu Ala Val Ser Leu Gly Gln Arg Ala Thr Ile Ser Cys Arg Ala Ser
35 40 45

Glu Ser Val Asp Ser Tyr Gly Asn Ser Phe Met His Trp Tyr Gln Gln
50 55 60

Lys Pro Gly Gln Pro Pro Lys Leu Leu Ile Tyr Arg Ala Ser Asn Leu
65 70 75 80

Glu Ser Gly Ile Pro Ala Arg Phe Ser Gly Ser Gly Ser Arg Thr Asp
85 90 95

Phe Thr Leu Thr Ile Asn Pro Val Glu Ala Asp Asp Val Ala Thr Tyr
100 105 110

Tyr Cys Gln Gln Ser Asn Glu Asp Pro Tyr Thr Phe Gly Gly Gly Thr
115 120 125

Lys Leu Glu Ile Lys Arg Gly Gly Gly Gly Ser Gly Gly Gly Gly Ser

130 135 140

Gly Gly Gly Gly Ser Gly Gly Gly Gly Ser Glu Val Gln Leu Lys Glu
145 150 155 160

Ser Gly Pro Gly Ile Leu Gln Pro Ser Gln Thr Leu Ser Leu Thr Cys
165 170 175

Ser Phe Ser Gly Phe Ser Leu Asn Thr Ser Gly Met Gly Val Gly Trp
180 185 190

Ile Arg Gln Pro Ser Gly Lys Gly Leu Glu Trp Leu Ala His Ile Trp
195 200 205

Trp Asp Asp Asp Lys Arg Tyr Asn Pro Ala Leu Lys Ser Arg Leu Thr
210 215 220

Phe Ser Lys Asp Thr Ser Ser Asn Gln Val Phe Leu Lys Ile Ala Ser
225 230 235 240

Val Asp Thr Ala Asp Thr Ala Thr Tyr Tyr Cys Val Arg Gly Gly Asn
245 250 255

Ser Phe Ala Tyr Trp Gly Gln Gly Thr Leu Val Thr Val Ser Thr Phe
260 265 270

Thr Cys Phe Val Val Gly Ser Asp Leu Lys Asp Ala His Leu Thr Trp
275 280 285

Glu Val Ala Gly Lys Val Pro Thr Gly Gly Val Glu Glu Gly Leu Leu
290 295 300

Glu Arg His Ser Asn Gly Ser Gln Ser Gln His Ser Arg Leu Thr Leu
305 310 315 320

Pro Arg Ser Leu Trp Asn Ala Gly Thr Ser Val Thr Cys Thr Leu Asn
325 330 335

His Pro Ser Leu Pro Pro Gln Arg Leu Met Ala Leu Arg Glu Pro Ala
340 345 350

Ala Gln Ala Pro Val Lys Leu Ser Leu Asn Leu Leu Ala Ser Ser Asp
355 360 365

Pro Pro Glu Ala Ala Asn Val Asn His Lys Pro Ser Asn Thr Lys Val
370 375 380

```
Asp Lys Lys Val Glu Pro Lys Ser Cys Asp Lys Thr His Thr Cys Pro
385                 390                 395                 400

Pro Cys Pro Ala Pro Glu Leu Leu Gly Gly Pro Ser Val Phe Leu Phe
                405                 410                 415

Pro Pro Lys Pro Lys Asp Thr Leu Met Ile Ser Arg Thr Pro Glu Val
            420                 425                 430

Thr Cys Val Val Val Asp Val Ser His Glu Asp Pro Glu Val Lys Phe
        435                 440                 445

Asn Trp Tyr Val Asp Gly Val Glu Val His Asn Ala Lys Thr Lys Pro
    450                 455                 460

Arg Glu Glu Gln Tyr Asn Ser Thr Tyr Arg Val Val Ser Val Leu Thr
465                 470                 475                 480

Val Leu His Gln Asp Trp Leu Asn Gly Lys Glu Tyr Lys Cys Lys Val
                485                 490                 495

Ser Asn Lys Ala Leu Pro Ala Pro Ile Glu Lys Thr Ile Ser Lys Ala
            500                 505                 510

Lys Gly Gln Pro Arg Glu Pro Gln Val Tyr Thr Leu Pro Pro Ser Arg
        515                 520                 525

Asp Glu Leu Thr Lys Asn Gln Val Ser Leu Thr Cys Leu Val Lys Gly
    530                 535                 540

Phe Tyr Pro Ser Asp Ile Ala Val Glu Trp Glu Ser Asn Gly Gln Pro
545                 550                 555                 560

Glu Asn Asn Tyr Lys Thr Thr Pro Pro Val Leu Asp Ser Asp Gly Ser
                565                 570                 575

Phe Phe Leu Tyr Ser Lys Leu Thr Val Asp Lys Ser Arg Trp Gln Gln
            580                 585                 590

Gly Asn Val Phe Ser Cys Ser Val Met His Glu Ala Leu His Phe Trp
        595                 600                 605

Val Leu Val Val Val Gly Gly Val Leu Ala Cys Tyr Ser Leu Leu Val
    610                 615                 620

Thr Val Ala Phe Ile Ile Phe Trp Val Arg Ser Lys Arg Ser Arg Leu
625                 630                 635                 640
```

```
Leu His Ser Asp Tyr Met Asn Met Thr Pro Arg Arg Pro Gly Pro Thr
                645                 650                 655

Arg Lys His Tyr Gln Pro Tyr Ala Pro Pro Arg Asp Phe Ala Ala Tyr
            660                 665                 670

Arg Ser Arg Arg Asp Gln Arg Leu Pro Pro Asp Ala His Lys Pro Pro
        675                 680                 685

Gly Gly Gly Ser Phe Arg Thr Pro Ile Gln Glu Glu Gln Ala Asp Ala
    690                 695                 700

His Ser Thr Leu Ala Lys Ile Arg Val Lys Phe Ser Arg Ser Ala Asp
705                 710                 715                 720

Ala Pro Ala Tyr Gln Gln Gly Gln Asn Gln Leu Tyr Asn Glu Leu Asn
                725                 730                 735

Leu Gly Arg Arg Glu Glu Tyr Asp Val Leu Asp Lys Arg Arg Gly Arg
            740                 745                 750

Asp Pro Glu Met Gly Gly Lys Pro Gln Arg Arg Lys Asn Pro Gln Glu
        755                 760                 765

Gly Leu Tyr Asn Glu Leu Gln Lys Asp Lys Met Ala Glu Ala Tyr Ser
    770                 775                 780

Glu Ile Gly Met Lys Gly Glu Arg Arg Arg Gly Lys Gly His Asp Gly
785                 790                 795                 800

Leu Tyr Gln Gly Leu Ser Thr Ala Thr Lys Asp Thr Tyr Asp Ala Leu
                805                 810                 815

His Met Gln Ala Leu Pro Pro
            820
```